# EUROPEAN PATENT APPLICATION

(11) **EP 1 522 856 A1**
(43) Date of publication of application: **13.04.2005**
(21) Application number: 03022498.4
(22) Date of filing: 09.10.2003
(51) Int. Cl.: G01N 33/68

(54) **Method for the detection of a neurological or psychiatric, demential disease, especially Alzheimer's disease by use of cholecystokinin (CCK)-molecules, corresponding substances and detection reagents**

(71) Applicant: BioVisioN AG, 30625 Hannover (DE)
(72) Inventor: Selle, Hartmut, 30459 Hannover (DE); Zucht, Hans-Dieter, 30539 Hannover (DE); Lamerz, Jens, 30169 Hannover (DE); Möhring, Thomas, 22761 Hamburg (DE)
(74) Representative: Kröncke, Rolf, Dr.

(57) **Abstract**

The invention comprises CCK-proteins and CCK-protein fragments or corresponding nucleic acid molecules and their determination in biological samples from individuals suffering from neurological or psychiatric, demential diseases, especially Alzheimer's disease. Amino acid sequences are processed by specific proteolysis and may comprise postranslational modifications. Alterations of the concentrations of these substances indicate the presence of a neurological or psychiatric, demential disease especially Alzheimer's disease. The detection of these substances is furthermore suitable for monitoring of clinical studies or monitoring the disease progression. These substances are also suitable for therapy and prophylaxis of neurological or psychiatric, demential diseases especially Alzheimer's disease.

## Description

### Field of the invention

The invention is related to methods to detect neurological or psychiatric, demential diseases or the detection of a predisposition for such disease, especially Alzheimer's disease. To accomplish this the concentration of Cholecystokinin (CCK) -molecules in body fluids or other kind of samples obtained from individuals is measured using tests therefor. In addition the invention relates to CCK-molecules suitable to determine the presence and/or the progression of these neurological or psychiatric, demential diseases.

The invention also relates to detection kits and detection reagents such as antibodies, nucleic acids etc., which are suitable to detect the corresponding CCK-molecules. Furthermore the invention comprises pharmaceutical compositions containing CCK-molecules, e.g. CCK-proteins (Seq.-ID 28), fragments of CCK-proteins such as the CCK-protein fragments represented by Seq.-ID 1 to 27, CCK-peptidomimetics, CCK specific antibodies or antibody fragments, nucleic acids coding for CCK-proteins or CCK-protein fragments or nucleic acids complementary to these nucleic acids, CCK antagonists or CCK agonist, etc. for use in therapy, diagnosis, prognosis or for prophylactic treatment of neurological or psychiatric, demential diseases, especially Alzheimer's disease. The invention furthermore relates to methods suitable for stratification of patients especially within clinical trials and screening methods to identify CCK-binding molecules such as CCK-receptors, CCK-agonists, CCK-antagonists or modulators of CCK-expression, CCK-function or CCK-activity.

### Background of the invention

### Neurological or psychiatric diseases

Neurological or psychiatric, demential diseases are increasingly becoming a problem in industrialized countries especially as the mean life expectancy increases. Most of these kind of diseases are incurable and about 65 % of these patients suffer from Alzheimer's disease (Morbus Alzheimer, AD). Besides Alzheimer's disease there are known several non-Alzheimer dementias such as vascular dementia, Lewy-Body dementia, Binswanger dementia as well as demential diseases which represent "side effects" of other diseases such as Pick's disease, Gerstmann-Straussler-Scheinger-disease, Huntington disease, Creuzfeld-Jakob disease, depression etc. Alzheimer's disease is a neurodegenerative disease primary characterized by a strongly impaired short term,memory and cognitive deficits. Morphologically Alzheimer's disease is associated with amyloid deposits (" senile plaques" ) and degenerated neuronal cells (" tangles" ) within the brain. These morphological changes can only be analyzed post mortem and so far are the only available clear proof for the presence of Alzheimer's disease.

### Diagnosis of Alzheimer's disease

Methods suitable for diagnosis of Alzheimer's disease in a living individual are neuro-psychological tests, such as the "Mini-Mental Status Examination" (MMSE) or imaging techniques such as magnetic resonance imaging (MRI), computer tomography (CT) or single photon emmission computer tomographie (SPECT). However, Clark et al. showed that these kind of tests are of limited value for the determination of the progression of Alzheimer's disease as there appear to be big variations in " Mini-Mental Scores" (MMS) [1]. Neuro-psychological tests like the MMS Examination are done only by specialists or by big clinical centers and consequently are not readily accessible to many physicians which is another major drawback of current diagnostic methods for Alzheimer's disease. There is a clear medical need for a reliable diagnostic marker for Alzheimer's disease which can replace or complement currently used diagnostic methods such as MMSE. A biochemical diagnostic test measuring CCK-molecules in for example body fluids or tissues samples of patients has clear advantages, e.g. to be accessible for every physician who can sent patient material to clinical laboratories.

### Need for diagnostic methods for Alzheimer's disease

Similar methods for diagnosis of Alzheimer's disease and related diseases are described in WO 02/090974, WO 02/082075 and WO 03/048775. However there is still a need for additional markers for Alzheimer's disease which might be suitable to confirm diagnostic results obtained with other test systems or which might increase the diagnostic precision for example by combination with other test systems or which might diagnose other sub-groups of patients. Additional markers might help to characterize biochemical pathways of these diseases or even might be suitable for therapy by supplementing the patient with these CCK-molecules or by removing these CCK-molecules from the patient's circulation or body tissue.

### Therapy of Alzheimer's disease

Currently there is no causal therapy available for treatment or cure of Alzheimer's disease. Only the symptoms of Alzheimer's disease are treated for example by addition of neurotransmitters such as acetylcholine or acetylcholine esterase inhibitors. There are also antioxidative agents, scavenger molecules, calcium channel blockers, anti-inflammatory molecules, inhibitors of secretases, anti-amyloid antibodies and the immunization against amyloid peptides in use for therapy of Alzheimer's disease. Nevertheless to date there is no causal therapy for Alzheimer's disease available.

The scope of the invention is to find additional, reliable biomarkers for neurological or psychiatric, demential diseases, especially Alzheimer's disease to improve the diagnostic options especially for early detection of these diseases. In addition the invention comprises these marker molecules as therapeutics for treatment of neurological or psychiatric, demential diseases, especially Alzheimer's disease. To solve this technical need the invention provides a method for testing for a neurological or psychiatric, demential disease, especially Alzheimer's disease, or a test for the prediction of a predisposition for such a disease.

### CCK-protein fragments in CSF of Alzheimer's disease patients

Surprisingly the concentration of certain amino acid sequences corresponding to the CCK-protein which are present especially in cerebrospinal fluid (CSF) of patients suffering from Alzheimer's disease is altered as compared to control samples. These CCK-amino acid sequences represent fragments of the CCK-protein or the complete CCK-protein itself and are suitable for laboratory testing. The CCK-protein fragments are not random fragments but fragments of the CCK-protein which originate as a consequence of processes occurring in biological systems. These fragments are different from random proteolytic fragments which are commonly obtained by for example adding proteases such as trypsin to an isolated protein or to a biological sample containing proteins
Tests, e.g. for laboratory use, determine the presence or concentration of at least one CCK-protein, CCK-protein fragment or of a corresponding nucleic acid in a biological sample derived from an individual. The sequence of CCK is available in the public database GeneBank under the accession no. NP_000720 for the CCK- amino acid sequence and under the accession no.NM_000729 for the CCK-nucleic acid sequence. The results of the test can be used by physicians for diagnosis. Another embodiment of the invention is the use of this laboratory test to diagnose neurological or psychiatric, demential diseases, especially Alzheimer's disease at an early stage, e.g. by diagnosis of " Mild Cognitive Impairment" (MCI) or to diagnose neurological or psychiatric, demential diseases different from Alzheimer's disease such as for example Lewy-Body-Dementia or vascular dementia.

### Therapeutic useful molecules

As the CCK-molecules included in the invention most likely are associated with the disease itself the invention also includes their use as therapeutics or as drug targets. The removal or addition of these molecules might be of benefit to an individual in need for therapy or prophylaxis of a neurological or psychiatric, demential disease, especially Alzheimer's disease.

### CCK - Biological background

Cholecystokinin (CCK) is synthesized by neuronal cells and I cells in the small intestinal mucosa as a 115 amino acid polypeptide. Plasma CCK most likely originates from I-cells of the small intestinal mucosa and CCK33, CCK22 and CCK58 belong to this group whereas CCK8 is primarily present in neuronal tissue [2]. In total neuronal CCK represents the majority of CCK present in the organism [2, 3]. There is known at least one polymorphism in CCK resulting in an altered amino acid sequence, namely amino acid 95 is changed from arginine to tryptophan and further polymorphisms possibly exist. CCK is altered by a number of postranslational processing steps, including the removal of a 20 amino acid N-terminal signal sequence, and the addition of sulfate groups to Tyrosin 97, 111 and 114, and carboxyamidation of the C-terminus (phenylalanine at position 103). Prior to carboxyamidation 12 C-terminal amino acids are removed. The resulting 83 amino acid long CCK molecule (CCK83) represents amino acids 21 to 103 of CCK [2, 3]. Other processing products, all including the C-terminus of CCK83, are the CCK peptides comprising 4, 5, 6, 7, 8, 12, 22, 33, 39, and 58 amino acids termed CCK4, CCK5, CCK6, CCK7, CCK8, CCK12, CCK22, CCK33, CCK39 and CCK58. These CCK-molecules are usually carboxyamidated and at least one tyrosine side chain bears a sulfate modification. Many of these CCK-molecules are generated from CCK-83 by processing at mono- or di-basic sites by prohormone convertases [2, 3] such as PC-1, PC-2, PC-5, PC-7, PACE4 or Furin. However some CCK-molecules such as CCK-5 and CCK-7 are obviously generated at least in part by other proteases different from prohormone convertases. In addition not all mono- and dibasic sites present in CCK are processed by prohormone convertases. In mice with a defect in the protease carboxypeptidase E there are present CCK8 molecules with additional amino acids at the C-terminus, e.g. which contain either Gly-Arg-Arg or just Gly termed CCKB-GRR and CCK8-G [4].

There are two receptors known for CCK-molecules, type A and type B CCK-receptors. Type B receptors (CCKB) are present in the central nervous system and recognize sulfatated and non-sulfatated CCK-molecules whereas type A receptors (CCKA) are present in the gastrointestinal tract as well as in certain brain regions and selectively recognize sulfated CCK-molecules. Both CCK-receptors mediate different functions. Type A receptors mediate gall bladder and muscle contraction, intestinal motility, pancreatic enzyme, insulin and hormone secretion, satiety and growth. Type B receptors mediate growth, histamine and pepsinogen secretion, inhibit dopamine release, support panic attacks and improve memory function [5, 6] .

### Definitions

### CCK-protein and CCK

As used herin the terms CCK and CCK-protein is definded by the complete amino acid sequence corresponding to the GeneBank accession number NP_000720, this molecule is termed CCK-protein or CCK within this patent application. Other accession numbers for the CCK-protein are AAA53094, GMHUCP, P06307, 1305270A, AAH08283, AAP35637, AAP36308 and P23362. Included in this definition are variants or mutants of the CCK-protein showing at least 70%, preferably 80%, preferably 90%, preferably 95%, preferably 99% homology to the GeneBank accession number NP_000720. The homology is calculated according to the description provided in the following paragraphs. These variants or mutants of CCK-proteins can be present in nature or can originate from non-natural sources. Natural sources can be CCK-proteins from different species, different alleles, different gene loci, etc. Non-natural sources can be CCK-proteins generated by recombinant technologies like for example site-directed mutagenesis or random mutagenesis, the latter of which can be achieved for example by use of chemicals, or by use of ionizing radiation. The corresponding altered nucleic acid mutants may include substitutions, deletions, insertations, invertations or combinations of these alterations. The alterations can be present in coding as well as in non-coding nucleic acid sequences such as for example promoter sequences, 3'- or 5'-untranslated sequences. Sequence alterations can be conservative, that means not changing the amino acid sequence, and they can be non-conservative, resulting in altered amino acid sequences. The resulting mutated CCK-proteins can be biologically active, partial biologically active or biologically inactive. Further included are CCK-proteins with and without signal sequences, processed, non-processed, soluble or transmembrane or membrane associated CCK-proteins. Also included are CCK-proteins resulting from alternative splicing, alternative translations start and stop positions, RNA editing, alternative postranslational modifications, especially CCK-proteins with sulfate groups, N-terminal pyroglutamate modifications, carboxyamidations, translation of stop codons into unusual amino acids such as seleno-cysteine or pyro-lysine as well as CCK-proteins originated through other mechanisms present in nature.

### CCK-protein fragments

By definition all CCK-protein fragments are fragments of a CCK-protein. Preferably these fragments are at least 8 amino acids long. CCK-protein fragments also share at least 70%, preferably 80%, preferably 90%, preferably 95%, preferably 99% homology to the corresponding fragments ot the sequence having GeneBank accession number NP_000720. The homology is calculated according to the description provided in the following paragraph "Homology of sequences" . Percentage of homology is calculated on the basis of the length of the CCK-protein fragment not on the basis of the length of the CCK-protein. Consequently, a CCK-protein fragment with a sequence length of 20 amino acids which includes 4 amino acids different from the corresponding sequence of the CCK-protein is a 80% homologue fragment of the CCK-protein.

### CCK-nucleic acids

Nucleic acids coding for CCK-proteins or CCK-protein fragments are regarded as being DNA, RNA and DNA-RNA hybrid molecules both of natural origin and prepared synthetically, enzymatically or by recombinant techniques. Nucleic acids of natural origin are present in public databases such as the NCBI database under the accession numbers AH002739, L00354, L29400, BC008283,BT006991 and BT007640. Included are the coding and the corresponding complementary strand of nucleic acids as well as single stranded, double stranded, linear or circular molecules of nucleic acids. These nucleic acid molecules may represent a part of larger nucleic acid molecules as used in molecular biology such as plasmids, cosmids, phage particles, artificial chromosomes, viral vectors, retroviruses, adenoviruses, adeno-like viruses, bacculoviruses, etc. Also included are nucleic acid molecules which are composed partially or completely of modified nucleotides which comprise modified nucleotides having for example high in vivo stability, such as, for example, phosphorothioates. The modification of the nucleotides for example can reside within the base entity, the sugar entity or the phosphor entity of the nucleotide. Such stabilized nucleic acids are already known and used in the art.

### CCK-molecules

The term " CCK-molecule" as used within this application is meant to include all CCK-proteins, CCK-protein fragments and CCK-nucleic acids as defined in the previous paragraphs.

### Calculation of sequence homologies

To determine the homology between amino acid or nucleic acid sequences several computer software packages are known in the art, such as the GCG package (Genetics Computer Group, University of Wisconsin, Madison, WI, USA), including GAP [7], BLASTP, BLASTN, FASTA [8] and the commonly known Smith-Waterman-algorithm. Preferred parameters for amino acid homology searches are the algorithms of Needleman und Wunsch [9], the BLOSUM 62 matrix [10], a Gap Penalty of 12, a Gap Length Penalty of 4 and a Threshold of Similarity of 0. These parameters can also be used with the GAP algorithm. The previous parameters are default parameters for amino acid sequence homology searches. Gaps at the ends of sequences do not reduce the value for their homology. If very short sequences are analyzed it may be necessary to increase the expectation value to 100000 and to reduce the word size down to a value of 2. Other algorithms such as gap opening penalties, gap extension penalties and other matrixes including those named in the software handbook, Wisconsin package, version 9, september 1997 may also be used for calculating the homology between sequences. The choice which algorithms and parameters are most suitable depends on the particular case. If only two sequences are compared for homology GAP or " Best Fit" are preferred algorithms, if one sequence is compared to a whole database of sequences the FASTA or BLAST algorithms are preferred. A homology value of 70% obtained using the described algorithms is named a 70% homology within this patent application. The same is true for higher or lower homology values.

### Chemically or post-translationally modified amino acid sequences

A chemically or post-translationally modified amino acid sequence may consist of both D- and of L-amino acids, and combinations of D- and L-amino acids. These sequences may additionally comprise modified/unusual amino acids, i.e. amino acids which do not belong to the 20 standard amino acids. Numerous examples of such amino acids are know in the art [11]. There are also numerous example of post-translational modifications known in the art such as phosphate- or sulphate-modifications, glycosylation, amidation, deamidation, pyroglutamate modifications, oxidized or amidated amino acid side chains etc.

### Spezifische Hybridisierung

The term " specifically hybridizing" refers to the association between nucleotide molecules of sufficiently complementary sequence to permit such hybridization under pre-determined conditions generally used in the art. The exact length of the nucleotide molecules suitable for hybridizing reactions will depend upon many factors, including temperature and reaction conditions used. For example, for diagnostic applications, depending on the complexity of the target sequence, the nucleotide molecules, such as a PCR-primers, typically contain 15 to 25 or more nucleotides, although they may contain fewer nucleotides. The nucleotide molecule sequence need not reflect the exact complementary sequence of the target nucleotide molecules. For example, a non-complementary nucleotide fragment may be attached to the 5' or 3' end of the nucleotide molecule, with the remainder of the nucleotide molecule sequence being complementary to the target nucleotide molecule.

Appropriate conditions enabling specific hybridization of nucleotide molecules of varying complementarity are well known in the art and are commonly determined empirically by using routine testing. As a starting point for instance, one common formula for calculating the stringency conditions required to achieve specific hybridization between nucleotide molecules in northern blots is set forth below (Sambrook, J., and D.W. Russell. 2001. Molecular Cloning - A Laboratory Manual. *Cold Spring Harbor Laboratory Press, Cold Spring Harbor, NY, USA.* 3rd Edition.)

Hybridization-temperature = 81.5 °C + 16.6 x Log [Sodium concentration present in the reaction] + 0.41 x (percentage of G and C nucleotides) - 0.63 x (percentage of formamide present in the reaction) - 600 / number of base pairs in duplex

As an illustration of the above formula, using a sodium concentration of 0.368 M and 50% formamide, with a GC content of 42% and an average probe size of 200 bases, the hybridization-temperature is 81.5 + 16.6 x Log 0.368 + 0.41 x 42 - 0,63 x 50 - 600/200 = 57 °C. The hybridization-temperature of a duplex decreases by 1 to 1.5 °C with every 1% decrease in sequence homology.

### Significance

In this patent application, an error probability of less than 10%, preferably less than 5% further preferably less than 1% is defined as significant.

### Sensitivity

Sensitivity is defined as the proportion of patients with the disease who acquire a positive diagnostic result in a diagnosis for the disease, i.e. the diagnosis correctly indicates the disease.

### Specificity

The specificity is defined as the proportion of healthy patients who acquire a negative diagnostic result in a diagnosis for the disease, i.e. the diagnosis correctly indicates that no disease is present.

### Embodiments of the invention

### Diagnostic methods

A preferred embodiment of the invention is the use of CCK-molecules for diagnosis and prognosis of neurological or psychiatric, demential diseases, especially Alzheimer's disease. For diagnostic purpose there are several strategies common and suitable:
a) Often it is possible to determine if a biomarker is present or absent in a biological sample and this indicates the absence or presence of the disease in the individual.
b) Using another strategy first the commonly present concentration range of the biomarker is determined in healthy and diseased individuals and a cut-off point is calculated distinguishing healthy from ill individuals. For each CCK-molecule there is an specific cut-off point. This cut-off point allows to clearly distinguish healthy from diseased individuals.
c) In still another strategy the concentration of a biomarker, such as a CCK-molecule, in a biological sample is compared relatively to the concentration of the same biomarker in a control sample. Significant changes in the concentration of the biomarker are declared as positive diagnostic result. The direction of the concentration change of each distinct biomarker molecule is fixed and does not change from one to another patient or individual.

Control samples eventually used for analysis may represent pool-samples comprising a mixture of individual control samples.

In another preferred embodiment a defined pattern of two or more different CCK-molecules can be used as marker panel for diagnosis or prognosis of a neurological or psychiatric, demential disease, especially Alzheimer's disease. The invention furthermore comprises the combination of the determination of at least one CCK-molecule with other known markers or diagnostic methods for these diseases to enable or to improve the diagnostic result.

In still another embodiment the invention includes the diagnosis of early stages of the disease the monitoring of disease progression or success of a therapeutic strategy, or the identification of subgroups of patients which are susceptible for a certain therapeutic strategy (stratification). Early stages of neurological or psychological diseases, especially Alzheimer's disease are "Mild Cognitive Impairment" (MCI), " Mini Mental Status" (MMS) scores below 27, or a set of tests including MMS as suggested by the "Consortium to Establish a Registry for Alzheimer's disease" (CERAD), "Global Deterioration Scale" (GDS) values above 1, " Clinical Dementia Rating Scale" (CDR) values above 0, etc. MMS scores are recorded in a range from 0 to 30 and a score of 27-30 is regarded as normal or healthy. GDS and CDR values are recorded for different cognitive abilities and for GDS the scale ranges from 1 to 7, with 1 regarded as normal or healthy, and for CDR the scale range is from 0 to 3 with 0 regarded as normal or healthy. Mild cognitive impairment is dependant on age and education and definitions used for MCI are determining memory fuction and other cognitive abilities using for example MMS-, GDS-, CDR- or CERAD-tests. A determined score which is at least 1 standard deviation below the score of that particular age and education matched test group indicates mild cognitive impairment [12].

### Suitable diagnostic markers

Preferred biomarker molecules are Seq.-ID 1 to Seq.-ID 29 shown in the sequence protocol. Their corresponding sequences are listed in Table 1. Furthermore, CCK-protein fragments of Seq.-ID 1 to Seq.-ID 28 are suitable as biomarker especially if they share at least 70%, preferably 80%, preferably 90%, preferably 95%, preferably 99% sequence homology to Seq.-ID 28 or to the sequence of a CCK-protein of the species of the individual tested. A further embodiment of the invention are CCK-protein fragments generated through proteolytic activity especially of prohormone convertases and carboxypeptidases. Also CCK-protein fragments comprising at least 8 amino acids with different lengths of additionally added C- and/or N-terminal added CCK-protein sequences are suitable as biomarker for a neurological or psychiatric, demential disease, especially Alzheimer's disease. Especially at least 8 amino acids long CCK-protein fragments comprising parts of the CCK-protein sequence from amino acid 1-49, or 41-81 or 64-95 or 97-115 are included in the invention. Other preferred embodiments of the invention are CCK-proteins or CCK-protein fragments containing point mutations resulting in at maximum two altered amino acids in the sequence.

Further preferred biomarkers are CCK-proteins or CCK-protein fragments with chemical, enzymatical or postranslational modifications. This results in, among others, altered molecular masses resulting in altered mass-spectrometric properties and altered chromatographic retention times for example during reverse phase chromatography. Especially the peptides may contain sulfate and phosphate modified side chains of amino acids, N-terminal or C-terminal amidations, N- or O-linked glycosylations, N-terminal pyroglutamate modifications and the peptides may be oxidized and contain for example Sodium-, Potassium-, Ammonium- or other adducts.

Another embodiment of the invention are nucleic acids coding for CCK-proteins or CCK-protein fragment as described above. Also included are nucleic acid sequences complementary to the coding nucleic acid sequences, which also can be used as markers for diagnosis of neurological or psychiatric, demential diseases, especially Alzheimer's disease.

Also included in the invention are salts of CCK-molecules.

### CCK-molecules generated by prohormoneconvertases

The protein sequence of CCK contains several di-basic sites which are potential processing sites for prohormone convertases such as PC-1 (also known as PC-3), PC-2, PC-4, PC-5 (also known as PC-6), PC-7/8, PACE4 or furin. Processing products originating from a CCK-protein or CCK-protein fragment through the action of a prohormone convertase often represent peptides with biological activity and are not just degradation products. Consequently, another embodiment of the invention are CCK-protein fragments originating from the sequence of the CCK-protein as a consequence of processing by prohormone convertases and carboxypeptidases. Prohormone convertases cut amino acid sequences C-terminal to di-basic and sometimes also mono-basic sites. A di- or mono-basic sites contains two or one basic amino acids, e.g. arginine or lysine. Consequently the CCK-protein sequence potentially can be cut by prohormone convertases between the following positions of the amino acid sequence: between amino acid 35/36, 42/43, 45/46, 49/50, 55/56, 64/65, 71/72, 77/78, 81/82, 91/92, 95/96 and 106/107. Subsequently to the action of prohormone convertases often the C-terminal basic amino acids are removed gradually by carboxypeptidases.

### Indirect detection of CCK-molecules

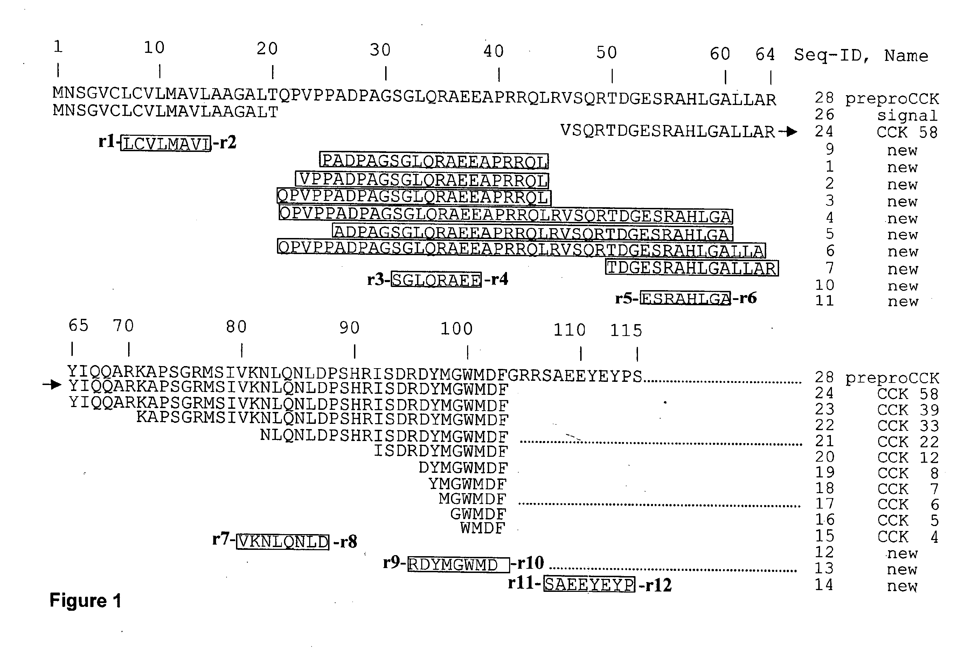

There are several CCK-protein fragments know in the art such as CCK83, CCK58, CCK39, CCK33, CCK22, CCK12, CCK8, CCK7, CCK6, CCK5 and CCK4. These CCK-protein fragments are generated in vivo through the proteolytic activity of certain enzymes such as prohormone convertases. In the course of the processing of CCK-proteins into CCK-protein fragments by-products may be generated. New by-products of proteolytic processing of CCK83 to CCK58 which are not know in the prior art are Seq.-ID 1 to Seq.-ID 4 as these four sequences represent most of the sequence between the signal sequence of the CCK-protein and the N-terminal end of the biological active CCK58 fragment (see figure 1). Correspondingly Seq.-ID 1 to Seq.-ID 7 represent new by-products of the processing of CCK83 to CCK39 (see figure 1). Furthermore Seq.-ID 7 represents a new by-product which is released during the processing of CCK58 to CCK39 (see figure 1). Consequently in another embodiment of the invention quantification of certain CCK-protein fragments such as for example Seq.-ID 1 to 4 can be used to indirectly determine the concentration of CCK58, quantification of certain CCK-protein fragments such as for example Seq.-ID 5 to 7 can be used to determine the concentration of CCK39 and quantification of certain CCK-protein fragments such as for example Seq.-ID 8 can be used to determine the conversion rate of CCK58 into CCK39.
Sequences of the CCK-protein fragments in one letter amino acid code:

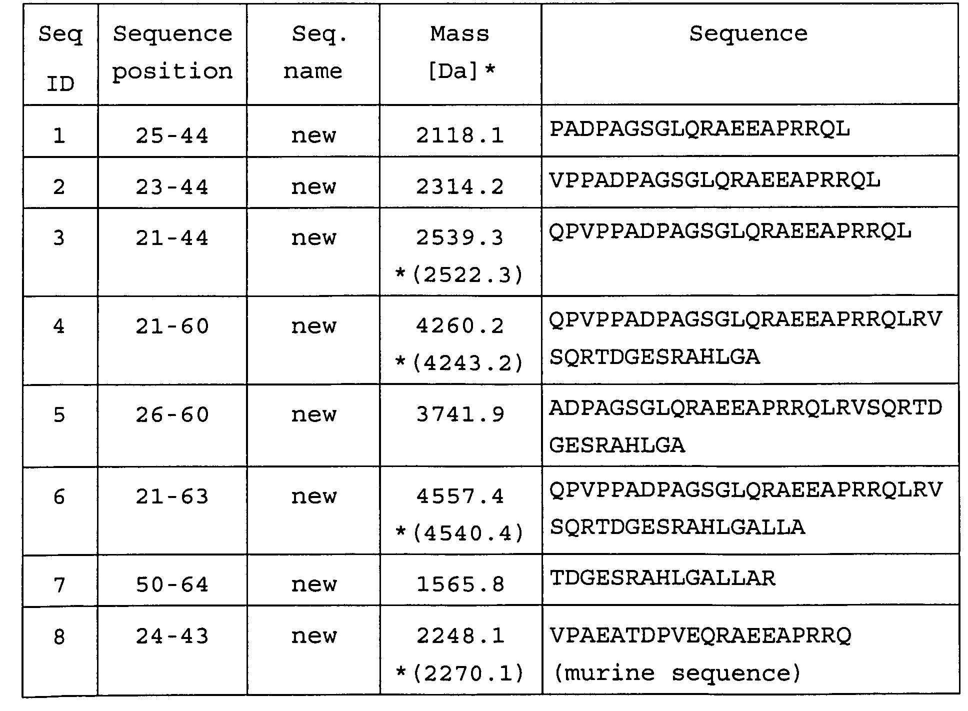

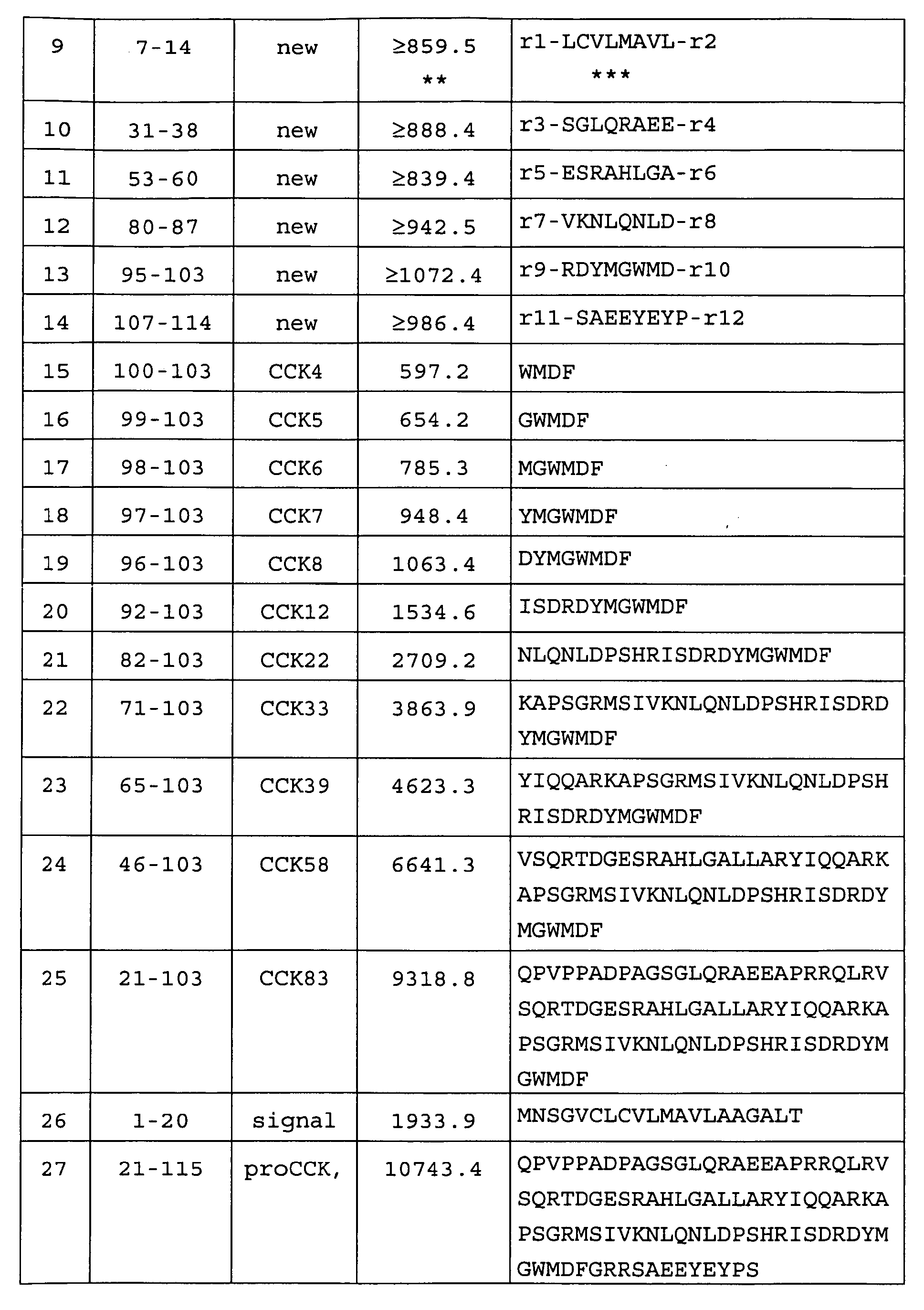

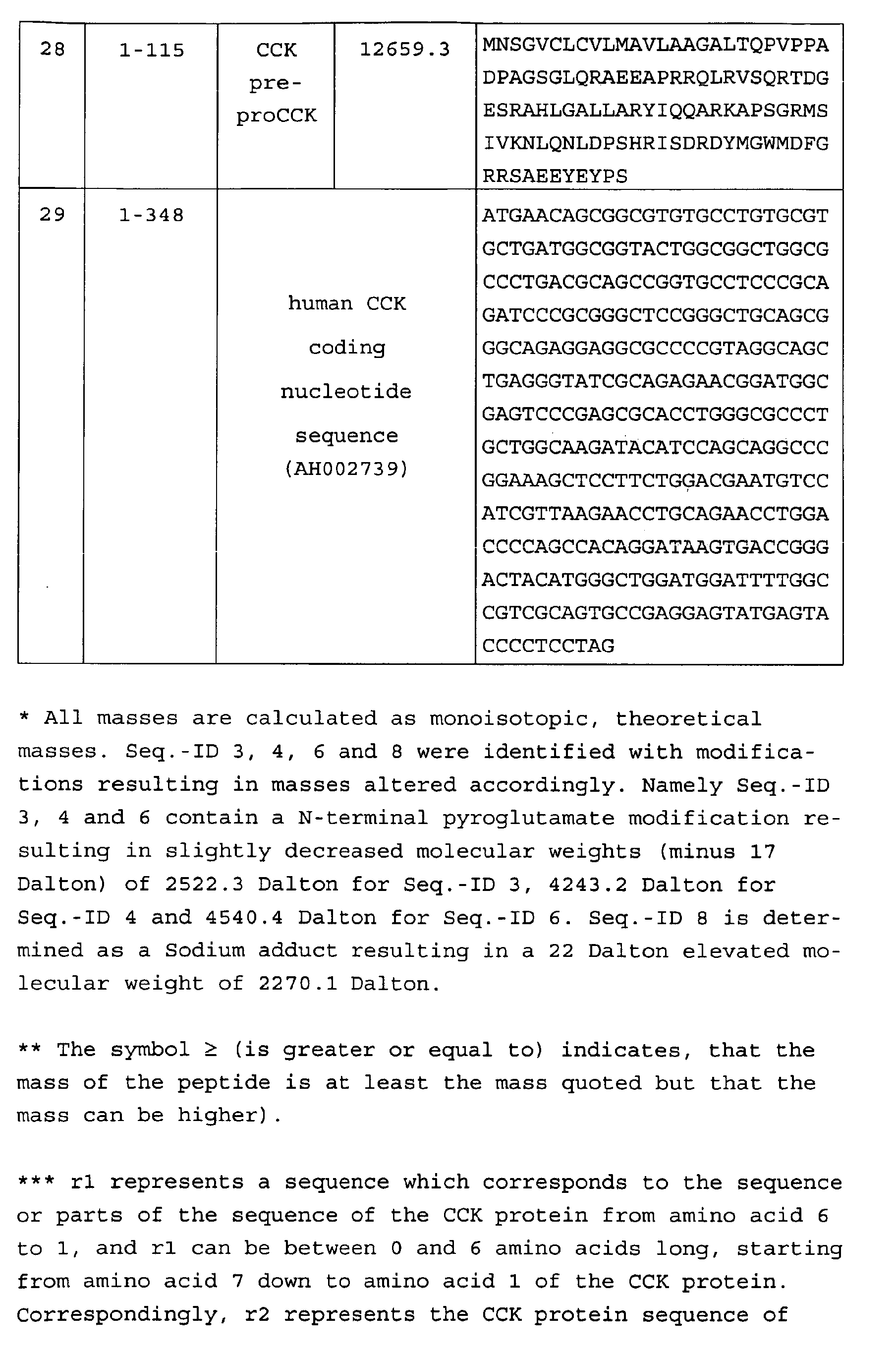

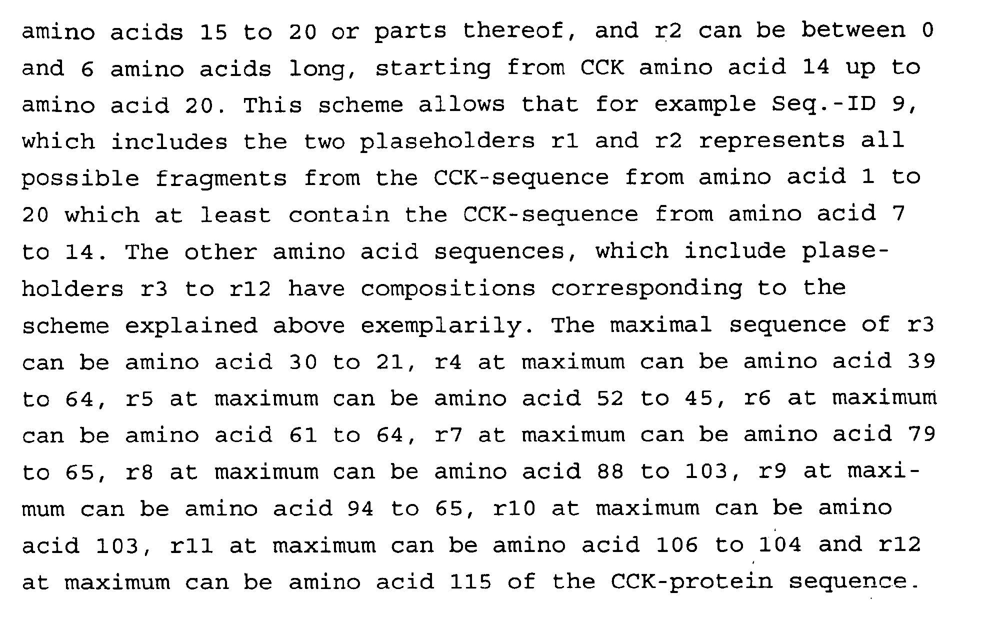

### Suitable biological samples

The biological sample may preferably be cerebrospinal fluid (CSF) or a sample such as cerebrospinal fluid, whole blood, blood products such as serum, plasma and blood cells, lymph fluid, urine, stool, tear fluid; saliva, synovial fluid, sputum, tear fluid, cell or tissue samples or cell or tissue homogenates etc. This depends inter alia on the sensitivity of the chosen detection method (mass spectrometry, ELISA etc.) and on the method itself. It is also possible, where appropriate, to use homogenized tissue samples, tissue sections and biopsy specimens. Therefore in a further embodiment of this invention tissue homogenates can be produced inter alia from human tissue samples such as biopsies. These tissues can be comminuted for example with manual homogenizers, with ultrasound homogenizers or with electrically operated homogenizers such as, for example, Ultraturrax, and then be boiled in a manner known to the skilled worker in acidic aqueous solutions with, for example, 0.1 to 0.2 M acetic acid for 10 minutes. The extracts are then subjected to the respective detection method, e.g. a mass-spectrometric investigation. The samples can be prepared, for example where appropriate diluted or concentrated, and stored in the usual way.

### Detection methods for CCK-molecules

### Physical detection methods

One embodiment of the invention is the use of physical methods which are able to indicate the peptides of the invention qualitatively or quantitatively. These methods include, inter alia, liquid chromatography, thin-layer chromatography, circular dichroism, CD spectroscopy, bio-chip technologies using nucleic acids, proteins, antibodies or other suitable binding or coupling agents (e.g. Ciphergen Biosystems, Inc., Fremont, CA, USA) as well as numerous different spectroscopic methods, which employ electromagnetic rays of various wave lengths, e.g. wave length from 1 nm to 1m. These methods include among others atom absorption spectroscopy, chemiluminescence spectrometry, electron spectrometry, X-ray spectrometry, infra red spectrometry, fourier transform IR spectrometry, Raman spectroscopy, laser spectrometry, " lochbrenn" spectrometry, luminescence spectrometry, plasma spectrometry, magnetic resonance spectroscopy (NMR), mass spectrometry, micro wave spectroscopy, " Mössbauer" spectrometry, fluorescence spectrometry, UV/visible-spectrometry etc. This entails comparison of quantitative measured results from a sample to be investigated with the measurements obtained in a group of patients suffering from neurological or psychiatric, demential diseases, preferably Alzheimer's disease, and a control group. It is possible to infer the presence of a neurological or psychiatric, demential diseases, in particular Alzheimer's disease, and/or the severity of this disease from these results.

### Reverse phase chromatography

A particularly preferred embodiment of this invention encompasses the use of reverse phase chromatography, in particular a C18 reverse phase chromatography column using mobile phases consisting of trifluoroacetic acid and acetonitrile, for separation of peptides in human cerebrospinal fluid. For example the fractions collected in each case each comprise 1/96 of the mobile phase volume used.

The fractions obtained in this way are analyzed with the aid of mass spectrometry, preferably with the aid of MALDI mass spectrometry (matrix-assisted laser desorption ionization) using a matrix solution consisting of, for example, L(-) fucose and alpha-cyano-4-hydroxycinnamic acid dissolved in a mixture of acetonitrile, water, trifluoroacetic acid and acetone. The presence of particular masses is established and the signal intensity quantified. These masses correspond to the masses of the amino acid sequences with the Seq.-IDs 1 to 28 of the invention.

### Precipitation and extraction methods

Another preferred embodiment of the invention is to perform a pre-fractionation of the sample by precipitation using precipitants such as ammonium sulfate, polyethylene glycol, trichloroacetic acid, acetone, ethanol etc. Other suitable precipitation methods are immune precipitations or precipitations initiated by alteration of physical factors such as temperature (heat precipitation) or pressure. Fractions obtained using these methods can be further analyzed individually or in groups. Another sample preparation method is the use of extraction methods using liquid solvents. One suitable solvent is a mixture of an organic solvent such as polyethylene glycol and an aqueous salt solution. Due to their physical properties substances present in the sample are enriched in certain fractions and subsequently can be analyzed with further methods.

### Mass spectrometry

A preferred embodiment of the invention is the determination of CCK-molecules by determination of their moleculare mass for example by use of mass spectrometry. In another preferred embodiment of the invention, the substances can be identified by MALDI (matrix-assisted laser desorption and ionization) mass spectrometry or a MALDI-TOF (Matrix-assisted-laser-desorption-and-ionisation-time-of-flight-) mass spectrometry. In this case, the mass-spectrometric determination further preferably includes at least one of the following mass signals, in each case calculated on the basis of the theoretical monoisotopic mass of the corresponding peptide. Slight differences from the theoretical monoisotopic mass are due to an experimental mass spectrometry error of a maximum of 500 ppm and due to the natural isotope distribution. In addition, in MALDI mass determinations a proton is added to the peptides owing to the method of measurement, whereby the mass increases by 1 Dalton. The following masses correspond to the theoretical monoisotopic masses of the peptides identified by us; calculated with suitable software, in this case GPMAW 4.02. These theoretical monoisotopic masses may occur singly or in combination in a sample: Seq-ID 1 = 2118.1 / Seq-ID 2 = 2314.2 / Seq-ID 3 = 2539.3 or 2522.3 / Seq-ID 4 = 4260.2 or 4243.2 / Seq-ID 5 = 3741.9 / Seq-ID 6 = 4557.4 or 4540.4 / Seq-ID 7 = 1565.8 / Seq-ID 8 = 2248.1 or 2270.1 / Seq-ID 9 ≥ 859.5 / Seq-ID 10 ≥ 888.4 / Seq-ID 11 ≥ 839.4 / Seq-ID 12 ≥ 942.5 / Seq-ID 13 ≥ 1072.4 / Seq-ID 14 ≥ 986.4 / Seq-ID 15 = 597.2 / Seq-ID 16 = 654.2 / Seq-ID 17 = 785.3 / Seq-ID 18 = 948.4 / Seq-ID 19 = 1063.4 / Seq-ID 20 = 1534.6 / Seq-ID 21 = 2709.2 / Seq-ID 22 = 3863.9 / Seq-ID 23 = 4623.3 / Seq-ID 24 = 6641.3 / Seq-ID 25 = 9318,8 / Seq-ID 26 = 1933.9/ Seq-ID 27 = 10743.4 and Seq-ID 28 = 12659.3 Dalton.

The symbol ≥ (is larger than or equal to) is to be understood to mean that the relevant peptides cannot have any larger masses but can have only the masses possible owing to the amino acids which are possibly additionally present at the ends of these peptides. Amino acids which may be additionally present at the ends of these peptides are not just any ones but only those which may be present at this sequence position owing to the sequence of the CCK-protein (Seq.-ID 28). The CCK-protein fragments with the Seq.-ID 3, 4 and 6 were identified with an N-terminal pyroglutamate modification, resulting in a mass reduction of about 17 Dalton and the CCK-protein fragment corresponding to Seq.-ID 8 was determined as a sodium adduct, resulting in an increased mass of about 22 Dalton. The masses including the modification of these peptide are for Seq.-ID 3 2522.3 Dalton, for Seq.-ID 4 4243.2 Dalton, for Seq.-ID 6 4540.4 Dalton and for Seq.-ID 8 2270.1 Dalton. For peptides identified with modifications both masses, connected with "or" , are shown. The second mass represents the mass of the modified peptide. It is possible, that these or other CCK-protein fragments may be present in nature with one or more other or additional modifications resulting in masses different to their theoretical masses as shown in table 1.

### Mass-spectrometric determination of the sequence of the substances

For the further practical application of this embodiment, further confirmation of the result of detection is advisable and possible by establishing the identity of the peptides corresponding to the masses, taking account exclusively of peptide signals which may be derived from a CCK-protein. This confirmation takes place by identifying the peptide signals preferably using methods of mass spectrometry, e.g. MS/MS analysis [13].

Novel, specific fragments of CCK-proteins were identified, and their significance was revealed by the method used for the invention.
The sequences of suitable CCK-proteins and CCK-protein fragments are indicated in the sequence listing Seq-ID 1 to 28. Some of these sequences represent novel substances which are not known in the prior art (Seq.-ID 1 to 14). The CCK-protein fragments of Seq.-ID 9 - 14 may comprise at the N- and/or C terminus additional amino acid sequences corresponding to the sequence of the CCK-protein at that particular position. The invention also encompasses the CCK-proteins, CCK-protein fragments and corresponding isolated nucleic acids prepared by recombinant techniques or synthetically, and isolated from biological samples, in unmodified, chemically modified or post-translational modified form.

### Molecular biologically detection techniques

The invention also encompasses nucleic acids which correspond to CCK-protein or CCK-protein fragments, and especially those which correspond to Seq.-ID 1 to 28 of the invention, the use thereof for the indirect determination and quantification of the relevant CCK-proteins and CCK-protein fragments. This also includes nucleic acids which represent, for example, non-coding sequences such as, for example, 5'- or 3' non-translated regions of the mRNA, or nucleic acids which show a sequence agreement with the CCK-nucleic acid sequence (Seq.-ID 29) which is sufficient for specific hybridization experiments and which are therefore suitable for the indirect detection of relevant CCK-proteins and CCK-protein fragments.

One exemplary embodiment thereof encompasses the obtaining of tissue samples, e.g. of biopsy specimens, from patients and the subsequent determination of the concentration of an RNA transcript corresponding to the sequence of the GeneBank accession No. NP_000729 or corresponding to a sequence showing at least 70% homology to NP_000729. This entails comparison of quantitative measured results (intensities) from a sample to be investigated with the measurements obtained in a group of patients suffering from Alzheimer's disease and a control group. Methods which can be used for the quantification are, for example, reverse transcriptase polymerase chain reaction (RT-PCR), quantitative PCR, real-time PCR (ABI PRISM® 7700 Sequence Detection System, Applied Biosystems, Foster City, CA, USA), in situ hybridization, Northern blots, nucleic acid chip assays and other methods known in the art. The presence or prognosis of a neurological or psychiatric, demential disease, preferably Alzheimer's disease and/or the severity thereof can be inferred from the results.

### Immunologic detection methods

In a further preferred embodiment of the invention, tests such as immunologic detection systems, preferably an ELISA (enzyme-linked immunosorbent assay) can be used to detect CCK-proteins and CCK-protein fragments. This immunologic detection picks up at least one CCK-protein or CCK-protein fragment. To increase the specificity, it is also possible and preferred to use a sandwich ELISA in which the detection of the CCK-proteins or CCK-protein fragments depends on the specificity of two antibodies which recognize different epitopes within the same molecule. However, other immunological diagnostic test systems such as direct or competitive ELISA, or other detection techniques such as, for example, RIA (radio immuno assay), EIA (enzyme immune assay), ELI-Spot assay (enzyme-linked immune spot assay), chemiluminescence assay, elektrochemiluminescence assay, flourecence assay, etc. are also suitable. CCK-proteins or CCK-proteins fragments isolated from biological samples, recombinant expressed or enzymatically prepared or chemically synthesized can be used as standard and/or control for the quantification.

Identification of these substances is generally possible for example with the aid of an antibody directed to that particular substance. Further methods suitable for such detections are, inter alia, Western blotting, immune precipitation, protein chip assays, Dot-Blots, plasmon resonance spectrometry (BIACORE® technology, Biacore International AB, Uppsala, Sweden), affinity matrices (e.g. ABICAP-Technologie, ABION Gesellschaft für Biowissenschaften und Technik mbH, Julich, Germany) etc. Substances/molecules suitable as detection agents are generally all those permitting the construction of a specific laboratory diagnostic detection system because they specifically bind a CCK-protein or a CCK-protein fragment. Numerous other immunologic detection systems also suitable for this purpose are known in the art.

### Use of the CCK-proteins and CCK-proteins fragments for producing diagnostic agents

The invention further comprises the use of at least one CCK-protein or CCK-protein fragment for the diagnosis of neurological or psychiatric, demential diseases, especially of Alzheimer's disease, and the use of CCK-proteins or CCK-proteins fragments for obtaining antibodies or other agents which, because of their CCK-specific binding properties, are suitable for developing diagnostic reagents for laboratory detection systems for these diseases. The invention furthermore comprises test kits containing CCK-proteins and/or CCK-protein fragments for use as standards. These test kits can also contain CCK-specific antibodies and/or antibody fragments immobilized on particles or on surfaces or prepared for immobilizing. The antibodies also can be labeled using enzymes, dyes, fluorescent dyes, radioactive substances, or other kind of labels enabling their use in diagnostic kits.

### Obtaining of CCK-proteins and CCK-protein fragments

A further embodiment of the invention is the obtaining of CCK-proteins and CCK-protein fragments using recombinant expression systems, in vitro translation, chromatographic methods and chemical synthesis protocols etc. which are known to the skilled worker. CCK-proteins and CCK-protein fragments can be isolated from natural sources or from recombinant expression systems by using reverse phase chromatography, affinity chromatography, ion exchange chromatography, gelfiltration, isoelectric focusing, preparative immune precipitation, ammonium sulfate precipitation, extraction using organic solvents, etc. The substances isolated to greater than 50% purity, preferably greater than 60%, preferably greater than 70%, preferably greater than 80%, preferably greater than 90% preferably greater than 95%, preferably greater than 99% can be used for therapy or prophylaxis of neurological or psychiatric, demential diseases, especially Alzheimer's disease, can be used as standards in diagnostic assays, can be used for the manufacture of antibodies specifically recognizing these substances etc. The CCK-proteins or CCK-protein fragments may be C- or N-terminally or internally fused to heterologous amino acid sequences such as poly-histidine sequences, hemagglutinin epitopes (HA-tag), HIV-Tat-sequences, VP22-sequences or proteins such as, for example, maltose-binding proteins, glutathione S-transferase (GST), green fluorescent protein (GFP) or protein domains such as the GAL-4 DNA binding domain or the GAL4 activation domain. The substances containing these additional amino acid sequences are for example suitable to isolate the substances, or to more conveniently detect these substances or to identify binding partners of these substances (using for example the yeast-two-hybrid system) or to enable the transfer of these substances from the extracellular to the intracellular space.

### Anti CCK antibodies

A preferred embodiment of the invention are antibodies or antibody fragments binding to CCK-proteins or CCK-protein fragments, especially antibodies binding to neo-epitopes of CCK-protein fragments. Furthermore a preferred embodiment of the invention is the production and isolation of antibodies or antibody fragments specific to CCK-proteins or CCK-protein fragments. A particularly preferred embodiment is the production of CCK-protein fragment-specific antibodies which recognize neo-epitopes, i.e. epitopes which are present only on CCK-protein fragments but not in a CCK-proteins. Such anti-CCK-protein fragment antibodies make the specific immunologic detection of CCK-protein fragments possible in the presence of CCK-protein.

Polyclonal antibodies can be produced by immunizations of experimental animals such as, for example, mice, rats, rabbits or goats. Monoclonal antibodies can be obtained for example by immunizations of experimental animals and subsequent application of hybridoma techniques or else via recombinant experimental approaches such as, for example, via antibody libraries such as the HuCAL® antibody library of MorphoSys, Martinsried, Germany, or other recombinant production methods known to the skilled worker. Antibodies can also be used in the form of antigen-binding antibody fragments such as, for example, F_{ab} fragments or F_{ab2} fragments etc. These antibodies or antibody fragments can also be part of fusion proteins or can be coupled to other proteins. Suitable fusion partners or proteins suitable for coupling are for example enzymes such as alkaline phosphatase or peroxidases or fluorescent proteins such as green fluorescent protein or toxins such as bacterial or fungal toxins. Alternatively antibodies or antibody fragments can be coupled to non-protein compounds such as fluorescent dyes for example FITC, BODIPY or Texas red, to radioactive isotopes such as phosphor, tritium, iodine, technetium, etc. or to other structures such as biotin or pharmacological active substances such as anti-cancer drugs, or anti microbial drugs, etc.

### Therapeutic methods

Concentrations of certain CCK-protein-fragments are clearly altered in Alzheimer's disease patients. Consequently supplementation or removal of CCK-molecules is a possible therapeutic option for treatment of patients suffering from neurological or psychiatric, demential diseases, especially Alzheimer's disease. The aim of this strategy is to adjust the in vivo concentrations of CCK-molecules to at least the concentrations present in healthy individuals. It might be of benefit for the individual to reverse the disease-related concentration change of CCK-molecules. For example if a certain CCK-molecule is present in decreased concentrations in an individual suffering form a neurological or psychiatric, demential disease, the concentration of that CCK-molecule can be increased even above the normal concentrations present in healthy individuals. If the molecule concentration is increased its concentration my be decreased below the normal concentration present in healthy individuals. In contrast it is also possible that in the patient the concentration of a CCK-molecule is increased and for therapeutic intervention it is suitable to further increase the concentration of this CCK-molecule, as the increased CCK-molecule concentration may be a biological response of the organism of the individual to cure itself. Consequently the opposite may also be true: a CCK-molecule is decreased and for therapeutic purpose the concentration of this CCK-molecule can be further decreased.

It is also possible, that the CCK-molecule used for diagnosis is different from the CCK-molecule used for therapy. A reason for this scenario is that for example a certain fragment of the whole CCK-molecule is suitable to detect the disease but for treatment of the disease the concentration of the complete CCK-molecule has to be altered. However, once a pair of disease marker and molecule suitable for the treatment of the corresponding disease is known this combination can be used for future diagnosis and therapy of that disease.

Another preferred embodiment of the invention is the combination of different therapeutic strategies to increase the therapeutic benefit for the individual in need of such a therapy.

### Decreasing CCK-molecule concentrations

Suitable tools for decreasing the concentration of CCK-molecules are for example neutralizing antibodies, antibody fragments, antibody-fusion proteins or other polypeptides or molecules binding to CCK-molecules such as CCK-molecule receptors. Other tools for decreasing the concentration of CCK-molecules are antisense nucleic acids, siRNA-nucleic acids (RNA-mediated interference, RNAi), peptide-nucleic acids (PNAs), ribozymes, triplex nucleic acids, etc. or antagonists of CCK-molecules or substances decreasing the expression, translation, postranslational modification or the processing of CCK-molecules or substances increasing the degradation of CCK-molecules such as proteases.

### Increasing CCK-molecule concentrations/half-lifes

Suitable tools to increase the concentration of CCK-molecules are the CCK-molecules itself (produced by recombinant, enzymatic chemical or other techniques or purified from natural sources), circular, linear, single or double stranded nucleic acids coding for CCK-molecules such as plasmids, cosmids, mRNA, cDNA, expression systems using retroviruses, adenoviruses, bacculoviruses, phages, yeast, bacteria, mammalian or plant cells, etc. Other tools to increase the concentration of CCK-molecules are the use of agonists of CCK-molecules, molecules increasing the expression, translation, postranslational modification, processing, half life (for example pegylation of polypeptides) of CCK-molecules, etc.

### CCK-molecule mimetics

CCK-molecules as well as modulators of the concentration of CCK-molecules can be replaced by mimetics or by polymers composed totally or in part of modified amino acids or modified nucleotides. These substances can be used for therapy of neurological or psychiatric, demential diseases, especially Alzheimer's disease.

### Therapy development and monitoring through CCK-protein or CCK-protein fragment determinations

A further exemplary use is the quantitative or qualitative determination of the abovementioned CCK-proteins or CCK-protein fragments for estimating the efficacy of a therapy under development for neurological or psychiatric, demential diseases, in particular Alzheimer's disease. The invention is also suitable for stratification of participants of clinical trials to test new therapies for treatment of neurological or psychiatric, demential diseases, in particular Alzheimer's disease. The testing of efficacy and the selection of the correct patients for therapies and for clinical trials is of outstanding importance for successful application and development of a therapeutic agent, and no clinically measurable parameter making this reliably possible is yet available for Alzheimer's disease [14].

### Examination of the therapeutic efficacy of CCK-proteins, CCK-protein fragments and of agents which modulate the expression and the bioavailability of these substances

One exemplary embodiment thereof encompasses the cultivation of cell lines and their exposure to CCK-proteins, CCK-protein fragments or with substances which promote the expression of CCK-proteins or CCK-protein fragments, or which promote the processing of CCK-proteins. Substances which promote processing are for example proteases such as prohormone convertases. It is possible thereby to establish the biological properties of CCK-proteins and CCK-protein fragments in connection with neurological or psychiatric, demential diseases, in particular Alzheimer's disease. Fusion molecules can also be used for the treatment of the cell lines, e.g. fusion proteins comprising sequences which enable for example the transport of fusion molecules from the extracellular space into the cell such as HIV-tat or VP22-sequences. Cell lines can also be transfected with inter alia expression vectors, mRNA or other nucleic acid molecules coding for molecules which directly or indirectly modulate the expression of CCK-protein or CCK-protein fragments. These modulating molecules can be prohormone convertases, antisense nucleic acids, siRNA-molecules, ribozymes, triplex-forming nucleic acids, CCK-agonists or -antagonists etc. Alternatively cell lines can be treated directly with anti-CCK antibodies, CCK-agonists or CCK-antagonists, with antisense-, ribozyme-, triplex-forming- or siRNA-nucleic acids etc. It is possible to treat a cell line with two or more different expression vectors, proteins or other molecules at the same time. Cell lines which appear suitable as neurological model systems in connection with CCK in particular can be used for such investigations.

Read-out systems which can be used for these investigations are inter alia tests which measure the rate of proliferation of the treated cells, their metabolic activity, the rate of apoptosis of the cells, changes in cell morphology, in the expression of cell-intrinsic proteins or reporter genes or which measure the release of cytosolic cell constituents as markers for cell death.
It is also suitable to measure the in vivo activity of these molecules employing experimental animals such as mice, rats, rabbits, dogs, apes etc., especially if these experimental animal strains are considered as model of neurological or psychiatric, demential diseases, in particular as model of Alzheimer's disease. Read-out parameters may be the survival time of the animals, their behavior and their short-term memory. One example of a memory test which is suitable for experimental animals is the Morris water maze test. Further parameters which can be used are the determination of body function such as body temperature, pulse rate, heart and lung function, blood pressure, brain currents, etc. Further test are measurements of neurological or metabolic mediators such as proteins, peptides, second messengers, hormones, lipids, steroids, etc. in samples such as blood, tissue samples, urine, cerebrospinal fluid etc. Furthermore morphological and histological investigations on tissues such as for example, the brain can be done.

Another method to investigate the function of CCK-proteins and CCK-protein fragments is the generation of experimental animals with CCK-deficiencies (knock outs) or with increased expression of CCK-proteins or CCK-protein fragments. This can be done with standard molecular biological methods as known in the art. The CCK-expression can be altered globally in the whole organism or only locally, depending on the experimental method used. Suitable experimental animals are Caenorhabditis elegans, drosophila, zebrafish, mice, rats, etc.

### Compositions containing CCK-molecules and CCK-modulating molecules

The invention further comprises the use of compositions containing substances such as CCK-proteins, CCK-protein fragments as well as the corresponding peptidomimetics for therapy of neurological or psychiatric, demential diseases, especially Alzheimer's disease. Peptidomimetics are molecules which have the activity of the corresponding peptide or protein but are not build from the standard set of 20 amino acids but from other structures such as for example beta-amino acids or spiegelmers® (Noxxon Pharma AG, Berlin, Germany) or other non-amino acid structures which can substitute amino acid structures. Agonist, antagonist, antibodies directed towards CCK-proteins or CCK-protein fragments are also suitable to modulate the concentration of CCK-proteins and CCK-protein fragments. Instead of whole antibodies the use of antibody fragments, antibody-fusion proteins or other molecules selectively binding to CCK-proteins or CCK-protein fragments is suitable for therapy or prophylaxis. Also nucleic acids coding for CCK-protein or CCK-protein fragments or complementary nucleic acid sequences may be used for therapy.

In another embodiment two or more CCK-proteins and CCK-protein fragments can be combined or can be coupled together or can be coupled with other non-CCK sequences. The coupling can be of covalent or non-covalent nature. The final molecule can be a linear, branched or circular molecule and combinations of covalently coupled, non-covalently coupled, linear, branched or circular molecule parts are possible. An example for a non-covalent coupling is a biotin-labeled protein such as CCK coupled to streptavidin-coupled antibodies mediated by binding of streptavidin to biotin.

A further embodiment of the invention comprises the use of compositions containing substances such as antisense nucleic acids, triplex-building nucleic acids, siRNA nucleic acids, ribozymes and other nucleic acids which are suitable to modulate the expression of CCK-proteins or CCK-protein fragments. Also suitable are agonists or antagonists of CCK-protein or CCK-protein fragment activities.

### Formulation of pharmaceutical compositions

Another embodiment of the invention are galenic formulations or chemical modifications of CCK-proteins, CCK-proteins fragments and their corresponding nucleic acids in such a way enabling or improving for example their transfer through the blood-brain-barrier and/or the blood-liquor-barrier or improving their transport through the cell membrane into the cytosol or improving their survival, stability, i.e. exhibiting an increased or decreased half-life in vivo or exhibiting an increased half-life at room temperature or below and in liquid or solid form, or their transfer into the circulation during their passage through the gastrointestinal tract, the urogenital tract, the lymphatic system, the subarachnoidal space, for topic application for inhaling or for direct injection onto tissues such as muscle, fat rissue or brain or for in vitro treatment of cells, etc. These properties render these modified CCK-proteins or CCK-proteins fragments particularly suitable for therapy of neurological or psychiatric, demential diseases especially Alzheimer's disease. Examples of such suitable modifications are the coupling of hydrocarbon chains such as polyethylenglycol to these molecules, the packaging of these molecules into liposomes or packaging of the substances into gastric acid resistant capsules enabling the passage of these substances through the stomach. Combining of CCK-sequences with foreign sequences such as the HIV-tat sequence can be used to improve the transport of these CCK-molecules into the intracellular space.

Another embodiment of the inventions comprises the addition of pharmacologically acceptable additives. Such additives can be preservative agents, sterile solvents, filler additives, dye additives, flavor additives, scent additives, etc.

A further embodiment of the invention is the combination of a CCK-molecule or a CCK-modulating substance with other medicaments. These other medicaments are preferably chosen from medicaments suitable for treatment of neurological diseases, especially Alzheimer's disease or chosen from medicaments suitable for treatment of diseases often associated with Alzheimer's disease or other neurological or psychiatric, demential diseases.

### Dosage of CCK-molecules for therapy

A further embodiment of the invention comprises dosage of these molecules via different routes, e.g. intravenous or subcutaneous injection, oral application, inhalable gas or aerosol, topic preparation or injection directly into the subarachnoidal cavity, into tissues such as muscle, fat, brain, etc. These different routes of application result in a higher bioavailability, higher biological activity or higher local concentration of these substances. For example proteins or protein fragments intended for oral application can be packed in acid resistant capsules which protect them from gastric acid and digestion during passage of the stomach. Highly hydrophobic substances can be converted to more hydrophilic substances by use of suitable galenic preparation methods rendering them more suitable for intravenous injections. For this purpose polymeric structures such as polyethylenglycol (PEG) can be coupled to these substances. Pegylated substances very often are better dissolved in aqueous solutions, are less immunogen and have a longer in vivo half-live. Pegylation also can improove the shelf life of substances at roomtemperature or at temperatures below roomtemperature such as 4°C, -20°C or -70°C, which are commonly used storage tempertures. Other dosage forms are packaging of the substances into polymers or gels (Atrix Labs, Fort Collins, CO, USA, Andrx Pharmaceuticals, Davie, FL, USA) etc. enabling their continuous and controlled release for longer periods of time.

### Screening methods

Another embodiment of the invention comprises methods to identify substances which modulate the expression, concentration or activity of CCK-proteins, CCK-protein fragments, CCK-agonists, CCK-antagonists, receptors binding to CCK-molecules, proteases which process CCK-proteins or CCK-protein fragments, or of nucleic acids coding for these substances. One example for a suitable screening method known in the art is contacting a biological sample with immobilized CCK-proteins or CCK-protein fragments. Subsequently substances which are present in the biological sample but did not bind to the immobilized CCK-proteins or CCK-protein fragments are removed by washing. The remaining substances which are bound to CCK-proteins or CCK-proteins fragments can be eluted by use of buffers changing physical parameters such as pH, ionic strength, hydrophobicity, temperature, etc. Substances recovered can be identified by standard methods such as protein sequencing, mass spectrometry, gel electrophoresis, bio chip assays, etc. Other test assays are for example phage display techniques, yeast-two hybrid assays, plasmon resonance spectrometry (Biacore International SA Uppsala, Sweden), reporter gene assays showing altered expression of CCK-proteins or CCK-protein fragments or showing altered biological activity of CCK-proteins or CCK-protein fragments etc. CCK-agonists or CCK-antagonists found using these screening methods can be used for therapy or prophylaxis of neurological or psychiatric, demential diseases especially Alzheimer's disease.

The invention is illustrated in detail below by means of examples. Reference is also made to the figures in this connection.
- Figure 1:: Alignment of CCK-protein fragments with the CCK protein, corresponding to the database accession No. NP_000720.
- Figure 2:: Reverse phase chromatogram of a separation and enrichment of CCK-protein fragments from cerebrospinal fluid
- Figure 3:: Mass-spectrum of a spectrometric measurement (MALDI) of a CCK-protein fragment, representing the sequence of the CCK-protein from amino acid 21 to 60 (Seq.-ID 4) as example
- Figure 4:: MALDI as relatively quantifying mass-spectrometric method
- Figure 5A-B:: MS/MS fragment spectra of the CCK-peptide fragment representing the CCK-sequence from amino acid 21 to 60 (Seq.-ID 4) as example
- Figure 6:: Box-whisker plot for quantitative comparison of the concentrations of the CCK-protein fragment representing the CCK-sequence from amino acid 21 to 60 (Seq.-ID 4) in samples from Alzheimer's disease patients compared with control samples.

Figure 1 shows an alignment of the CCK-protein sequence with the CCK-protein fragments according to Seq.-ID 1 to 26. Sequenced which are not known in the prior art are framed. Sequences known in the prior art are Seq.-IDs 15 to 26 representing CCK83, CCK58, CCK39, CCK33, CCK22, CCK12 and CCK8, CCK7, CCK6, CCK5, CCK4 and the signal sequence of CCK.

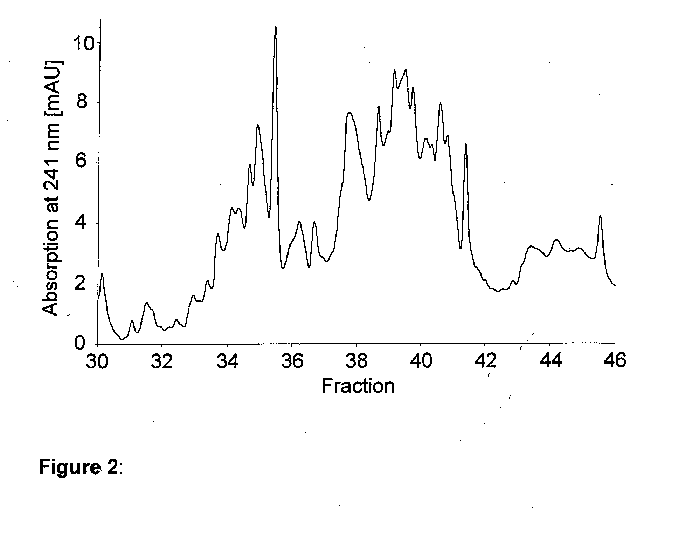

Figure 2 shows a chromatogram recorded using reverse phase chromatography as in example 2 for the separation and enrichment of the CCK-proteins and CCK-protein fragments from cerebrospinal fluid.

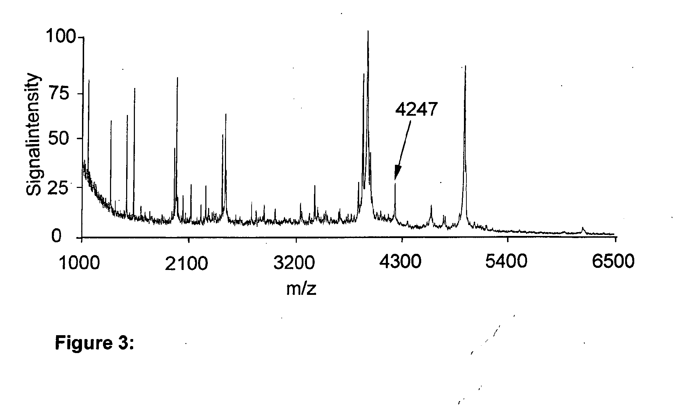

Figure 3 shows a spectrum resulting from MALDI mass-spectrometric measurement as in example 3 of the CCK-peptide fragment representing the CCK-sequence from amino acid 21 to 60 (Seq.-ID 4), with a theoretical monoisotopic mass of 4243.2 Dalton (mass of Seq.-ID 4 with pyroglutamate modification), after reverse phase chromatography of human cerebrospinal fluid as in Example 2.

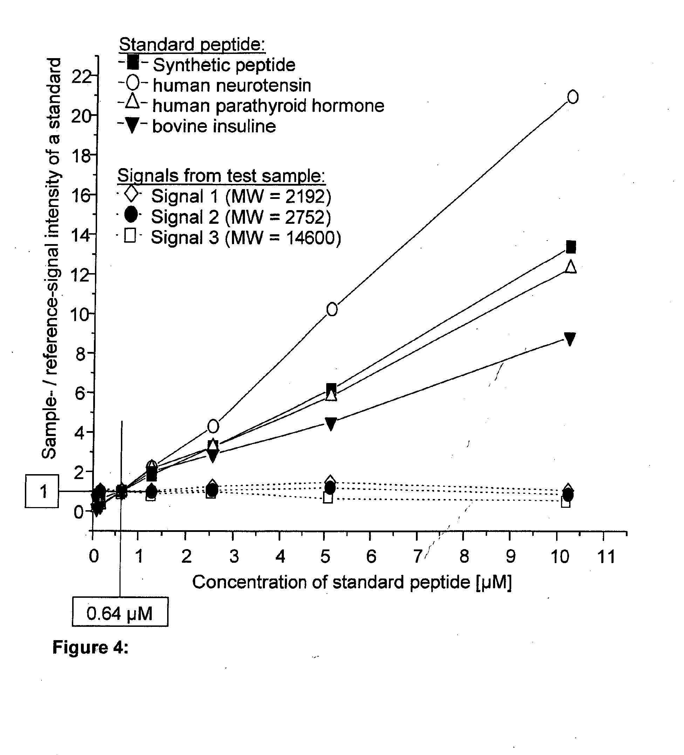

Figure 4 shows data generated by MALDI mass-spectrometric measurment as relatively quantifying mass spectrometry (MS) method. A sample was mixed with various amounts of different standard peptides, and the intensity both of these standard signals and of representative sample signals was measured. All signal intensities of the standards were standardized to their signal intensity at a concentration of 0.64 µM (= 1). Each peptide shows an individual typical ratio of signal intensity to concentration, which can be read off in this diagram from the slope of the plot.

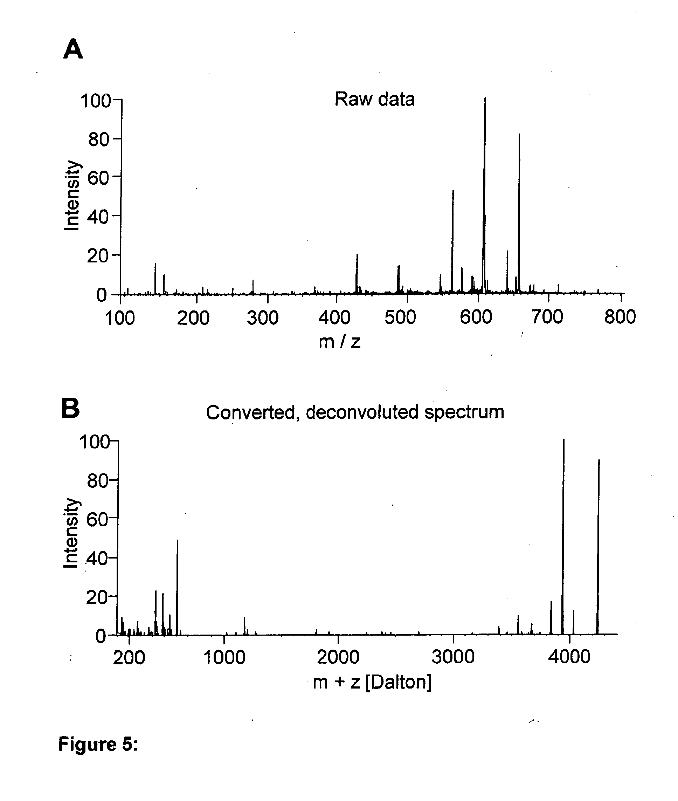

Figure 5 shows an MS/MS fragment spectrum as in example 4 of the CCK-peptide fragment representing the CCK-sequence from amino acid 21 to 60 (Seq.-ID 4) with a pyroglutamate modification.
- Upper trace:: raw data of the measurement.
- Lower trace:: converted, deconvoluted mass spectrum of the CCK-protein fragment.

The peak pattern is characteristic for the CCK-protein fragment corresponding to the CCK sequence of amino acid 21 to 60 (Seq.-ID 4) with a pyroglutamate modification.

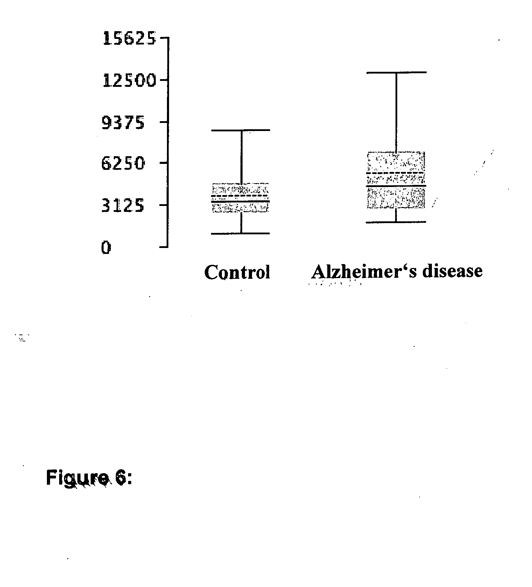

Figure 6 shows in the form of a box-whisker plot a comparison of the integrated MALDI mass-spectrometric signal intensities of the CCK-protein fragment corresponding to the CCK sequence from amino acid 21 to 60 with a pyroglutamate modification (Seq.-ID 4) in controls, compared with the signal intensities in samples from Alzheimer's disease patients.

### Example 1: Obtaining cerebrospinal fluid for determining CCK-protein fragments

CSF or cerebrospinal fluid (fluid of the brain and spinal cord) is the fluid which is present in the four ventricles of the brain and in the subarachnoid space and which is produced in particular in the choroid plexus of the lateral ventricle. Cerebrospinal fluid is usually collected by lumbar puncture and less often by suboccipital puncture or ventricular puncture. In lumbar puncture (spinal puncture) the puncture involves penetration of the spinal subarachnoid space between the 3rd and 4th or the 4th and 5th lumbar spinous vertebral with a long hollow needle, and thus CSF being obtained. The sample is then centrifuged at 2000× g for 10 minutes, and the supernatant is stored at -80°C.

### Example 2. Separation of peptides from cerebrospinal fluid (CSF) for mass-spectrometric measurement of CCK-protein fragments

For the detection of CCK-proteins and CCK-protein fragments in CSF by mass spectrometry, it is necessary in this example to separate the peptide constituents. This sample pretreatment serves to concentrate the peptides of the invention and to remove components which may interfere with the measurement. The separation method carried out is a reverse phase chromatography. Various RP chromatography resins and eluants are equally suitable for this. The separation of CCK-proteins and CCK-protein fragments using a C18 reverse phase chromatography column with the size of 4 mm × 250 mm supplied by Vydac is shown by way of the example below. Mobile phases of the following composition were used: mobile phase A: 0.06% (v/v) trifluoroacetic acid, mobile phase B: 0.05% (v/v) trifluoroacetic acid, 80% (v/v) acetonitrile. Chromatography took place at 33°C using an HP ChemStation 1100 supplied by Agilent Technologies with a micro flow cell supplied by Agilent Technologies. Human cerebrospinal fluid was used as sample. 440 µl of CSF were diluted with water to 1650 µl, the pH was adjusted to 2-3, the sample was centrifuged at 18000× for 10 minutes and finally 1500 µl of the sample prepared in this way were loaded onto the chromatography column. The chromatography conditions were as follows: 5% mobile phase B at time 0 min, from time 1 to 45 min continuous increase in the mobile phase B concentration to 50%, from time 45 to 49 min continuous increase in the mobile phase B concentration to 100% and subsequently up to time 53 min constant 100% buffer B. Collection of 96 fractions each of 0.5 ml starts 10 minutes after the start of the chromatography. The chromatogram of a cerebrospinal fluid sample prepared under the experimental conditions described herein is depicted in Figure 2.

### Example 3: Measurement of masses of peptides by means of MALDI mass spectrometry

For mass analysis, typical positive ion spectra of peptides were produced in a MALDI-TOF (matrix-assisted laser desorption/ionization time-of-flight) mass spectrometer. Suitable MALDI-TOF mass spectrometers are manufactured by PerSeptive Biosystems Framingham (Voyager-DE, Voyager-DE PRO or Voyager-DE STR) or by Bruker Daltonik Bremen (BIFLEX). The samples are prepared by mixing them with a matrix substance which typically consists of an organic acid. Typical matrix substances suitable for peptides are 3,5-dimethoxy-4-hydroxycinnamic acid, α-cyano-4-hydroxycinnamic acid and 2,5-dihydroxybenzoic acid. A lyophilized equivalent obtained by reverse phase chromatography corresponding to 500 µl of human cerebrospinal fluid is used to measure the CCK-protein fragments of the invention. The chromatographed lyophilized sample is dissolved in 15 µl of a matrix solution. This matrix solution contains, for example, 10 g/L α-cyano-4-hydroxycinnamic acid and 10 g/L L(-)fucose dissolved in a solvent mixture consisting of acetonitrile, water, trifluoroacetic acid and acetone in the ratio 49:49:1:1 by volume. 0.3 µl of this solution is transferred to a MALDI carrier plate, and the dried sample is analyzed in a Voyager-DE STR MALDI mass spectrometer from PerSeptive Biosystems. The measurement takes place in linear mode with delayed extraction™. An example of a measurement of one of the CCK-protein fragments of the invention is shown in Figure 3.

The MALDI-TOF mass spectrometer can be employed to quantify peptides such as, for example, CCK-proteins and CCK-protein fragments of the invention if these peptides are present in a concentration which is within the dynamic measurement range of the mass spectrometer, thus avoiding detector saturation. This is the case for the measurement of the CCK-proteins and CCK-protein fragments of the invention in cerebrospinal fluid at a CSF equivalent concentration of 33.3 µl per µl of matrix solution. There is a specific ratio between measured signal and concentration for each peptide, which means that the MALDI mass spectrometry can preferably be used for the relative quantification of peptides. This situation is depicted in Figure 4. If various amounts of different standard peptides are added to a sample, it is possible to measure the intensity both of these standard signals and of the sample signals. Figure 4 shows by way of example a MALDI measurement as relatively quantifying mass-spectrometric method. All signal intensities of the standards were standardized to their signal intensity at a concentration of 0.64 µM (= 1). Each peptide shows an individual, typical ratio of signal strength to concentration, which can be read off from the slope of the plot.

### Example 4: Mass spectrometric identification of the CCK-protein fragments

For quantification of the CCK-protein fragments of the invention it is necessary to ensure that the mass signals to be analyzed in fact relate to the CCK-proteins or CCK-protein fragments of the invention.

The peptides of the invention are employed for example using nanoSpray-MS/MS [13]. This entails a CCK-protein fragment ion being selected in the mass spectrometer on the basis of its specific m/z (mass/charge) value in a manner known to the skilled worker. This selected ion is then fragmented by supplying collision energy with an collision gas, e.g. helium or nitrogen, and the resulting fragments of the CCK-protein fragment are detected in the mass spectrometer in an integrated analysis unit, and corresponding m/z values are determined (principle of tandem mass spectrometry) [15]. The fragmentation behavior of peptides makes unambiguous identification of the CCK-protein fragments of the invention possible. If the accuracy of mass is, for example, 50 ppm it is possible to use computer-assisted search methods [16] for search sequences of CCK-protein fragments in sequence databases into which the sequence of a CCK-protein has been entered. In this specific case, the mass-spectrometric analysis took place with a Quadrupol-TOF Instrument, QStar-Pulsar model from Applied Biosystems-Sciex, USA. Examples of MS/MS fragment spectra are shown in Figure 5.

### Example 5: Mass spectrometric quantification of the CCK-protein fragments to compare their relative concentration in control samples with their concentration in patients' samples

A sample preparation as in Example 1 and 2 followed by a MALDI measurement of the CCK-protein fragments of the invention as in Example 3 were carried out on 279 clinical samples representing 86 passive (healthy) controls, 66 active controls (non-Alzheimer dementias) and 127 samples from patients suffering from Alzheimer's disease. An example of MALDI signal intensities is depicted in the form of a box-whisker plot in Figure 6. Samples were measured in the course of 4 independent measurement serieses to enable cross validation of the results. The box-whisker plot depicted makes it possible to compare the integrated MALDI mass-spectrometric signal intensities of the CCK-protein fragment in controls with the MALDI signal intensities in samples from Alzheimer's disease patients. The box, i.e. the columns in the diagram in Figure 6, include the range of MALDI signal intensities in which 50% of the respective MALDI signal intensities are found. The lines starting from the box and pointing upward and downward (whiskers) indicate the range in which in each case the 25% of measurements which show the highest signal intensities (upper quarter) are found, and in which the 25% of measurements which show the lowest signal intensities (lower quarter) are found. The full line in the columns indicates the median and the broken line in the columns indicates the mean.

The headings in this document are intended merely to provide structure to the text. They are not intended to limit or restrict the matters described. All the examples are intended to characterize the concept of the invention in more detail but are not intended to restrict the equivalence range of the invention.

In case this patent contains a term or phrase which is not clear to the skilled worker of that particular field or which is not clear in the context of the description provided by this patent the following references shall provide the definition for that term or phrase. In case there are different definitions for one term found in different references, the definition found in the reference citated first in the following list should be taken. The following references are citated for this purpose:
- The Merck Manual of Diagnosis and Therapy [17]
- Molecular Cloning - A Laboratory Manual [18]
- Current Protocols in Immunology [19]
- Current Protocols in Protein Science [20]
- Current Protocols in Pharmacology [21]
- Current Protocols in Cell Biology [22]

### Literatur

1. Clark, C.M., L. Sheppard, G.G. Fillenbaum, D. Galasko, J.C. Morris, E. Koss, R. Mohs, and A. Heyman. 1999. Variability in annual Mini-Mental State Examination score in patients with probable Alzheimer disease: a clinical perspective of data from the Consortium to Establish a Registry for Alzheimer's Disease. *Arch Neurol.* 56:857-62.
2. Rehfeld, J.F., G. Sun, T. Christensen, and J.G. Hillingso. 2001. The predominant cholecystokinin in human plasma and intestine is cholecystokinin-33. *J Clin Endocrinol Metab.* 86:251-8.
3. Rehfeld, J.F., I. Lindberg, and L. Friis-Hansen. 2002. Increased synthesis but decreased processing of neuronal proCCK in prohormone convertase 2 and 7B2 knockout animals. *J Neurochem.* 83:1329-37.
4. Beinfeld, M.C. 2003. Biosynthesis and processing of pro CCK: recent progress and future challenges. *Life Sci.* 72:747-57.
5. Wank, S.A. 1998. G protein-coupled receptors in gastrointestinal physiology. I. CCK receptors: an exemplary family. *Am J Physiol.* 274:G607-13.
6. Sebret, A., I. Lena, D. Crete, T. Matsui, B.P. Roques, and V. Dauge. 1999. Rat hippocampal neurons are critically involved in physiological improvement of memory processes induced by cholecystokinin-B receptor stimulation. *J Neurosci.* 19:7230-7.
7. Devereux, J., P. Haeberli, and O. Smithies. 1984. A comprehensive set of sequence analysis programs for the VAX. *Nucleic Acids Res.* 12:387-95.
8. Altschul, S.F., W. Gish, W. Miller, E.W. Myers, and D.J. Lipman. 1990. Basic local alignment search tool. *J Mol Biol.* 215:403-10.
9. Needleman, S.B., and C.D. Wunsch. 1970. A general method applicable to the search for similarities in the amino acid sequence of two proteins. *J Mol Biol.* 48:443-53.
10. Henikoff, S., and J.G. Henikoff. 1992. Amino acid substitution matrices from protein blocks. *Proc Natl Acad Sci U S A*. 89:10915-9.
11. Garavelli, J.S., Z. Hou, N. Pattabiraman, and R.M. Stephens. 2001. The RESID Database of protein structure modifications and the NRL-3D Sequence-Structure Database. *Nucleic Acids Res.* 29:199-201.
12. Bischkopf, J., A. Busse, and M.C. Angermeyer. 2002. Milde cognitive impairment - a review of prevalence, incidence and outcome according to current approaches. *Acta Psychiatr Scand.* 106:403-414.
13. Wilm, M., and M. Mann. 1996. Analytical properties of the nanoelectrospray ion source. *Anal Chem.* 68:1-8.
14. Engelborghs, S., and P.P. De Deyn. 2001. Biological and genetic markers of sporadic Alzheimer's disease. *Acta Med Okayama.* 55:55-63.
15. Papayannopoulos, I.A. 1995. The interpretation of collision-induced dissociation tandem mass spectra of peptides. *Mass Spectrom Rev*:49-73.
16. Perkins, D.N., D.J. Pappin, D.M. Creasy, and J.S. Cottrell. 1999. Probability-based protein identification by searching sequence databases using mass spectrometry data. *Electrophoresis.* 20:3551-67.
17. Beers, M.H., and R. Berkow. 1999. The Merck Manual of Diagnosis and Therapy. *Merck Research Laboratories, Whitehous Station*, *NJ, USA.* 17th Edition.
18. Sambrook, J., and D.W. Russell. 2001. Molecular Cloning - A Laboratory Manual. *Cold Spring Harbor Laboratory Press, Cold Spring Harbor, NY, USA.* 3rd Edition.
19. Coligan, J.E., A.M. Kruisbeek, D.H. Margulies, and E.M. Shevach. 2002. Current Protocols in Immunology. *John Wiley & Son, Inc. , Hoboken, NJ, USA.*
20. Coligan, J.E., B.M. Dunn, H.L. Ploegh, D.W. Speicher, and P.T. Wingfield. 2002. Current Protocols in Protein Science. *John Wiley & Son, Inc., Hoboken, NJ, USA.*
21. Enna, S.J., M. Williams, J.W. Ferkany, T. Kenakin, R.D. Porsolt, and J.P. Sullivan. 2002. Current Protocols in Pharmacology. *John Wiley & Son, Inc., Hoboken, NJ, USA.*
22. Bonifacino, J.S., M. Dasso, J. Lippincott-Schwartz, J.B. Harford, and K.M. Yamada. 2002. Current Protocols in Cell Biology. *John Wiley & Son, Inc. , Hoboken, NJ, USA.*

## Claims

1. A method for detection or prognosis of a neurological or psychiatric, demential disease or a predisposition to such a disease in a mammal or a human by determining a CCK-molecule.

2. The method according to claim 1, wherein the CCK-molecule detected is at least one amino acid sequence selected from the group consisting of:
a) Seq.-ID 1 to 28;
b) a fragment of Seq.-ID 1 to 28;
c) Seq.-ID 1 to 28 or a fragment thereof sharing at least 70% sequence homology to the corresponding sequence fragment of Seq.-ID 28;
d) a fragment generated by processing of Seq.-ID 28 at mono- or di-basic sites by proteolytic action of a prohormone convertase;
e) a fragment originating from amino acids 1 to 49, 41 to 81, 64 to 95 or 99 to 115 of Seq.-ID 28 with a sequence length of at least 8 amino acids;
f) a mutant of a substance according to claim 2a) to 2e) with a maximum of two amino acids differing from the corresponding amino acid sequence;
g) a chemically, enzymatically or postranslationally modified substance according to any one of the claims 2a) to 2f) preferably a substance with a sulfate group or an N-terminal pyroglutamate modification or a carboxyamidation; and/or
h) a nucleic acid sequence encoding any one of the above amino acid sequences.

3. The method according to claim 1 or 2, wherein
a) a specific reduction or a specific increase of the CCK-molecule is determined and
b) a significant change of the concentration of the CCK molecule is regarded as positive laboratory test result for detection or prognosis or predisposition of the disease.

4. A method according to any of claims 1 to 3 for diagnosing early stages of the disease in particular mild cognitive impairment.

5. A method according to any of claims 1 to 3 for identification of subgroups of individuals with neurological or psychiatric, demential diseases which are responding to certain therapy strategies.

6. A method according to any of claims 1 to 3 in combination with other diagnostic methods to increase the sensitivity and/or specificity of the diagnosis.

7. A method according to any of claims 1 to 6, wherein the biological sample used for diagnosis is cerebrospinal fluid, whole blood, blood products such as serum, plasma and blood cells, lymph fluid, urine, stool, tear fluid, saliva, synovial fluid, sputum, tear fluid, cell or tissue samples or a cell or tissue homogenate.

8. A method according to any of claims 1 to 7, wherein
a) a chromatographic separation is done prior to the identification, preferably a reverse phase chromatography and/or
b) a precipitation or extraction of the sample is done prior to the identification.

9. The method according to claim 1 to 8, whereby the determination of the substance is done using an activity assay, immunologic assay, molecular-biological assay, physical assay or chemical assay.

10. The method according to claim 9, whereby a physical determination of the substance is done by determining the mass of the substances, preferably by using mass spectrometry.

11. The method according to claim 10, whereby the determination of the mass at least comprises one of the following theoretical, monoisotopic masses from 2118.1 / 2314.2 / 2539.3 / 2522.3 / 4260.2 / 4243.2 / 3741.9 / 4557.4 / 4540.4 / 1565.8 / 2248.1 / 2270.1 / ≥ 859.5 / ≥ 888.4 / ≥ 839.4 / ≥ 942.5 / ≥ 1072.4 / ≥ 986.4 / 597.2 / 654.2 / 785.3 / 948.4 / 1063.4 / 1534.6 / 2709.2 / 3863.9 / 4623.3 / 6641.3 / 9318,8 / 1933.9 / 10743.4 and 12659.3 Dalton.

12. The method according to claim 9, whereby an immunologic assay preferably an ELISA, a radio immuno assay, a protein chip assay or a western blot is used for determination of the substance.

13. The method according to claim 9, whereby a molecular biological assay preferably a northernblot, a nucleic acid chip assay, a reverse-transcriptase PCR, a real-time PCR or a quantitative PCR is used for determination of the substance.

14. An amino acid which is:
a) Seq.-ID 1 to 11 and 14;
b) a fragment of Seq.-ID 1 to 11 and 14 which comprises at least 8 amino acids which are identical in type and in succession, compared to Seq.-ID 28;
c) a homolog of Seq.-ID 1 to 11 and 14 sharing at least 70% sequence homology to the corresponding sequence fragment of Seq.-ID 28;
d) a fragment originating from amino acids 1 to 49, 41 to 81, 64 to 95 or 99 to 115 of Seq.-ID 28 with a sequence length of at least 8 amino acids;
e) a mutant of an amino acid according to claim 14e) or 14f) with a maximum of two amino acids differing from the corresponding amino acid sequence;
f) a chemical or enzymatically or postranslational modified substance corresponding to any one of the claims 14a) to 14e) preferably a substance with a sulfate group or an N-terminal pyroglutamate modification or a carboxiamidation;
g) a fusion peptide or fusion protein comprising a sequence according to 14a) to 14f) and a second amino acid sequence which facilitates the detection or which enables the transport of the fusion molecules into the cell; and/or
h) a peptidomimetic substance of at least one of the substances according to 14a) to g).

15. A nucleic acid which is:
a) a nucleic acid coding for a substance according to claim 14;
b) a nucleic acid complementary to the nucleic acid sequence of a) or specifically hybridizing thereto; and/or
c) a ribozyme, an antisense nucleic acid, a triplex-forming nucleic acid, an siRNA nucleic acid or another substance specifically binding to one of the nucleic acids according to a) or b) or to CCK-mRNA corresponding to Seq.-ID 29 thereby inactivating the substance bound to.

16. A nucleic acid according to claim 15 which is a linear or circular, nucleic acid containing additional nucleic acid sequences besides nucleic acids according to claim 15a) to 15c), preferably sequences of an expression vector, a cloning vector, a viral vector, a phage vector or a yeast vector.

17. A nucleic acid according to claim 16 comprising at least one of the following elements: a promoter sequence, an antibiotic resistance sequence, a poly-A site, a translation start codon, a translation stop codon a multiple cloning sequence, a reporter gene, a sequence suitable for yeast-two hybrid interaction, a sequence suitable for phage display, a tag-sequence fused to the sequence of interest preferably enabling detection, purification or labeling to the resulting fusion product.

18. An antibody or a fragment of an antibody which is capable of binding to a substance according to claim 14a) to 14f).

19. A test kit for a method according to claim 1 to 13, whereby the test kit at least comprises a CCK-molecule or an antibody or an antibody fragment binding to a CCK-molecule and the CCK-molecule, antibody or antibody fragment is immobilized or labeled or is present in a state enabling immobilizing or labeling.

20. A pharmaceutical composition for therapy, diagnosis or prophylaxis comprising at least one substance or composition according to claim 14 to 18.

21. A pharmaceutical composition according to claim 20 additionally containing
a) another pharmaceutical active substance, preferably a pharmaceutical active substance suitable to treat, diagnose or prevent a neurological or psychiatric, demential disease especially Alzheimer's disease;
b) in addition a means of transport, preferably a transport peptide, preferably HIV-Tat, polymers, preferably polyethylenglycol or gastric acid resistant capsules or liposomes; and/or
c) at least one additional pharmacological acceptable additive.

22. The use of at least one substance according to claim 2 or 14 to 18 for the preparation of a pharmaceutical composition for use in diagnosis, therapy, or prophylaxis of a neurological or psychiatric, demential disease, preferably Alzheimer's disease.

23. The use according to claim 22, wherein the pharmaceutical composition further contains
a) another pharmaceutical active substance, preferably a pharmaceutical active substance suitable to treat, diagnose or prevent a neurological or psychiatric, demential disease especially Alzheimer's disease;
b) in addition a means of transport, preferably a transport peptide, preferably HIV-Tat, polymers, preferably polyethylenglycol or gastric acid resistant capsules or liposomes; and/or
c) at least one additional pharmacological acceptable additive.

24. The use of a substance according to claim 14 to 17 for the generation, production and purification of antibodies or for the development of clinical assays for the detection of neurological or psychiatric, demential diseases, preferably Alzheimer's disease.

25. A screening assay to identify:
a) substances able to modulate the expression of at least one substance according to claim 14 to 15;
b) receptors binding to at least one of the substances according to claim 14; and/or
c) agonists or antagonists of at least one substance according to claim 14.


SEQUENCE LISTING

<110>  BioVisioN AG

<120>  Method for the detection of a neurological or psy-chiatric, demential
disease, especially Alzheimer's disease by use of cholecystokinin (CCK)
molecules, corresponding sub-stances and detection reagents.

<130>  CCK

<160>  29

<170>  PatentIn version 3.1

<210>  1
<211>  20
<212>  PRT
<213>  Homo sapiens

<400>  1

Pro Ala Asp Pro Ala Gly Ser Gly Leu Gln Arg Ala Glu Glu Ala Pro
1               5                   10                  15

Arg Arg Gln Leu
            20

<210>  2
<211>  22
<212>  PRT
<213>  Homo sapiens

<400>  2

Val Pro Pro Ala Asp Pro Ala Gly Ser Gly Leu Gln Arg Ala Glu Glu
1               5                   10                  15

Ala Pro Arg Arg Gln Leu
            20

<210> 3
<211> 24
<212> PRT
<213> Homo sapiens

<400> 3

Gln Pro Val Pro Pro Ala Asp Pro Ala Gly Ser Gly Leu Gln Arg Ala
1               5                   10                  15

Glu Glu Ala Pro Arg Arg Gln Leu
            20

<210> 4
<211> 40
<212> PRT
<213> Homo sapiens

<400> 4

Gln Pro Val Pro Pro Ala Asp Pro Ala Gly Ser Gly Leu Gln Arg Ala
1               5                   10                  15

Glu Glu Ala Pro Arg Arg Gln Leu Arg Val Ser Gln Arg Thr Asp Gly
            20                  25                  30

Glu Ser Arg Ala His Leu Gly Ala
        35                  40

<210> 5
<211> 35
<212> PRT
<213> Homo sapiens

<400> 5

Ala Asp Pro Ala Gly Ser Gly Leu Gln Arg Ala Glu Glu Ala Pro Arg
1               5                   10                  15

Arg Gln Leu Arg Val Ser Gln Arg Thr Asp Gly Glu Ser Arg Ala His
            20                  25                  30

Leu Gly Ala
        35

<210>  6
<211>  43
<212>  PRT
<213>  Homo sapiens

<400>  6

Gln Pro Val Pro Pro Ala Asp Pro Ala Gly Ser Gly Leu Gln Arg Ala
1               5                   10                  15

Glu Glu Ala Pro Arg Arg Gln Leu Arg Val Ser Gln Arg Thr Asp Gly
            20                  25                  30

Glu Ser Arg Ala His Leu Gly Ala Leu Leu Ala
        35                  40

<210>  7
<211>  15
<212>  PRT
<213>  Homo sapiens

<400>  7

Thr Asp Gly Glu Ser Arg Ala His Leu Gly Ala Leu Leu Ala Arg
1               5                   10                  15

<210> 8
<211> 20
<212> PRT
<213> Mus musculus

<400> 8

Val Pro Ala Glu Ala Thr Asp Pro Val Glu Gln Arg Ala Glu Glu Ala
1               5                   10                  15

Pro Arg Arg Gln
            20

<210> 9
<211> 8
<212> PRT
<213> Homo sapiens

<400> 9

Leu Cys Val Leu Met Ala Val Leu
1               5

<210> 10
<211> 8
<212> PRT
<213> Homo sapiens

<400> 10

Ser Gly Leu Gln Arg Ala Glu Glu
1               5

<210> 11
<211> 8
<212> PRT
<213> Homo sapiens

<400>  11

Glu Ser Arg Ala His Leu Gly Ala
1               5

<210>  12
<211>  8
<212>  PRT
<213>  Homo sapiens

<400>  12

Val Lys Asn Leu Gln Asn Leu Asp
1               5

<210>  13
<211>  8
<212>  PRT
<213>  Homo sapiens

<400>  13

Arg Asp Tyr Met Gly Trp Met Asp
1               5

<210>  14
<211>  8
<212>  PRT
<213>  Homo sapiens

<400>  14

Ser Ala Glu Glu Tyr Glu Tyr Pro
1               5

<210>  15
<211>  4

<212>  PRT
<213>  Homo sapiens

<400>  15

Trp Met Asp Phe
1

<210>  16
<211>  5
<212>  PRT
<213>  Homo sapiens

<400>  16

Gly Trp Met Asp Phe
1               5

<210>  17
<211>  6
<212>  PRT
<213>  Homo sapiens

<400>  17

Met Gly Trp Met Asp Phe
1               5

<210>  18
<211>  7
<212>  PRT
<213>  Homo sapiens

<400>  18

Tyr Met Gly Trp Met Asp Phe
1               5

<210>  19
<211>  8
<212>  PRT
<213>  Homo sapiens

<400>  19

<210>  20
<211>  12
<212>  PRT
<213>  Homo sapiens

<400>  20

Ile Ser Asp Arg Asp Tyr Met Gly Trp Met Asp Phe
1               5                   10

<210>  21
<211>  22
<212>  PRT
<213>  Homo sapiens

<400>  21

Asn Leu Gln Asn Leu Asp Pro Ser His Arg Ile Ser Asp Arg Asp Tyr
1               5                   10                  15

Met Gly Trp Met Asp Phe
            20

<210>  22
<211>  33
<212>  PRT
<213>  Homo sapiens

<400>  22

Lys Ala Pro Ser Gly Arg Met Ser Ile Val Lys Asn Leu Gln Asn Leu
1               5                   10                  15

Asp Pro Ser His Arg Ile Ser Asp Arg Asp Tyr Met Gly Trp Met Asp
            20                  25                  30

Phe

<210>  23
<211>  39
<212>  PRT
<213>  Homo sapiens

<400>  23

Tyr Ile Gln Gln Ala Arg Lys Ala Pro Ser Gly Arg Met Ser Ile Val
1               5                   10                  15

Lys Asn Leu Gln Asn Leu Asp Pro Ser His Arg Ile Ser Asp Arg Asp
            20                  25                  30

Tyr Met Gly Trp Met Asp Phe
        35

<210>  24
<211>  58
<212>  PRT
<213>  Homo sapiens

<400>  24

Val Ser Gln Arg Thr Asp Gly Glu Ser Arg Ala His Leu Gly Ala Leu
1               5                   10                  15

Leu Ala Arg Tyr Ile Gln Gln Ala Arg Lys Ala Pro Ser Gly Arg Met
            20                  25                  30

Ser Ile Val Lys Asn Leu Gln Asn Leu Asp Pro Ser His Arg Ile Ser
        35                  40                  45

Asp Arg Asp Tyr Met Gly Trp Met Asp Phe
    50                  55

<210>  25
<211>  83
<212>  PRT
<213>  Homo sapiens

<400>  25

Gln Pro Val Pro Pro Ala Asp Pro Ala Gly Ser Gly Leu Gln Arg Ala
1               5                   10                  15

Glu Glu Ala Pro Arg Arg Gln Leu Arg Val Ser Gln Arg Thr Asp Gly
            20                  25                  30

Glu Ser Arg Ala His Leu Gly Ala Leu Leu Ala Arg Tyr Ile Gln Gln
        35                  40                  45

Ala Arg Lys Ala Pro Ser Gly Arg Met Ser Ile Val Lys Asn Leu Gln
    50                  55                  60

Asn Leu Asp Pro Ser His Arg Ile Ser Asp Arg Asp Tyr Met Gly Trp
65                  70                  75                  80

Met Asp Phe

<210> 26
<211> 20
<212> PRT
<213> Homo sapiens

<400> 26

Met Asn Ser Gly Val Cys Leu Cys Val Leu Met Ala Val Leu Ala Ala
1               5                   10                  15

Gly Ala Leu Thr
            20

<210> 27
<211> 95
<212> PRT
<213> Homo sapiens

<400> 27

Gln Pro Val Pro Pro Ala Asp Pro Ala Gly Ser Gly Leu Gln Arg Ala
1               5                   10                  15

Glu Glu Ala Pro Arg Arg Gln Leu Arg Val Ser Gln Arg Thr Asp Gly
            20                  25                  30

Glu Ser Arg Ala His Leu Gly Ala Leu Leu Ala Arg Tyr Ile Gln Gln
        35                  40                  45

Ala Arg Lys Ala Pro Ser Gly Arg Met Ser Ile Val Lys Asn Leu Gln
    50                  55                  60

Asn Leu Asp Pro Ser His Arg Ile Ser Asp Arg Asp Tyr Met Gly Trp

65                  70                  75                  80

Met Asp Phe Gly Arg Arg Ser Ala Glu Glu Tyr Glu Tyr Pro Ser
                85                  90                  95

<210>  28
<211>  115
<212>  PRT
<213>  Homo sapiens

<400>  28

Met Asn Ser Gly Val Cys Leu Cys Val Leu Met Ala Val Leu Ala Ala
1               5                   10                  15

Gly Ala Leu Thr Gln Pro Val Pro Pro Ala Asp Pro Ala Gly Ser Gly
            20                  25                  30

Leu Gln Arg Ala Glu Glu Ala Pro Arg Arg Gln Leu Arg Val Ser Gln
        35                  40                  45

Arg Thr Asp Gly Glu Ser Arg Ala His Leu Gly Ala Leu Leu Ala Arg
    50                  55                  60

Tyr Ile Gln Gln Ala Arg Lys Ala Pro Ser Gly Arg Met Ser Ile Val
65                  70                  75                  80

Lys Asn Leu Gln Asn Leu Asp Pro Ser His Arg Ile Ser Asp Arg Asp
                85                  90                  95

Tyr Met Gly Trp Met Asp Phe Gly Arg Arg Ser Ala Glu Glu Tyr Glu
            100                 105                 110

Tyr Pro Ser
        115

<210>  29
<211>  348
<212>  DNA
<213>  Homo sapiens

<400>  29
atgaacagcg gcgtgtgcct gtgcgtgctg atggcggtac tggcggctgg cgccctgacg      60

cagccggtgc ctcccgcaga tcccgcgggc tccgggctgc agcgggcaga ggaggcgccc     120

cgtaggcagc tgagggtatc gcagagaacg gatggcgagt cccgagcgca cctgggcgcc     180

ctgctggcaa gatacatcca gcaggcccgg aaagctcctt ctggacgaat gtccatcgtt     240

aagaacctgc agaacctgga ccccagccac aggataagtg accgggacta catgggctgg     300

atggattttg gccgtcgcag tgccgaggag tatgagtacc cctcctag                  348